# EUROPÄISCHE PATENTSCHRIFT

(11) **EP 3 597 113 B1**
(45) Veröffentlichungstag und Bekanntmachung des Hinweises auf die Patenterteilung: **17.01.2024**
(21) Anmeldenummer: 19179101.1
(22) Anmeldetag: 07.06.2019
(51) Int. Cl.: A61B 10/02, A61B 18/02, A61B 17/34

(54) **BIOPSATBERGEEINRICHTUNG**
BIOPSY MATERIAL RECOVERY DEVICE
DISPOSITIF DE RÉCUPÉRATION DU MATÉRIEL BIOPSIÉ

(30) Priorität: 17.07.2018 EP 18183972
(43) Veröffentlichungstag der Anmeldung: 22.01.2020
(73) Patentinhaber: Erbe Elektromedizin GmbH, 72072 Tübingen (DE)
(72) Erfinder: BRODBECK, Achim, 72555 Metzingen (DE); BLOBEL, Lars, 72119 Ammerbuch-Entringen (DE); KRONENTHALER, Jörg, 72145 Hirrlingen (DE); STÄBLER, Thomas, 72070 Tübingen (DE)
(74) Vertreter: Rüger Abel Patentanwälte PartGmbB

(56) Entgegenhaltungen:
- WO-A2-2008/044044
- US-A1- 2005 197 595
- US-A1- 2007 123 798
- US-A1- 2010 016 847
- US-A1- 2010 152 609
- US-A1- 2016 206 295

## Beschreibung

Die Erfindung betrifft eine Kryobiopsiesonde mit einer Biopsatbergeeinrichtung zum Bergen eines Biopsats.

Aus DE 10 2007 020 582 A1 ist ein kryochirurgisches Instrument und Verfahren zum Abtrennen einer Gewebeprobe bekannt, welches Instrument eine Sonde mit einem Sondenkopf zum Festfrieren einer Probe aufweist sowie eine Stützeinrichtung aufweist, in der die Sonde geführt ist. Mittels der Stützeinrichtung ist das umliegende Gewebe während des Abtrennens der Gewebeprobe abstützbar. Der Sondenkopf kann beim Abreißen der festgefrorenen Gewebeprobe in die Stützeinrichtung zurückgezogen werden, so dass die Gewebeprobe dadurch sicher in der Stützeinrichtung untergebracht ist.

Aus DE 10 2008 026 635 A1 ist eine Kryobiopsiesonde bekannt, welche durch einen Stützschlauch führbar ist. Der Stützschlauch und die Sonde können relativ zueinander beschleunigt werden, um die Gewebeprobe mit einer definierten Abrisskraft abzureißen.

Aus der WO 2008/044044 A2 ist ein als Nadel ausgebildetes Instrument bekannt, das als Stanzbiopsienadel, als Saugnadel oder als vakuumunterstütztes Biopsiesystem ausgebildet ist. An der Spitze der Stanzbiopsienadel ist eine flexible Hülle angeordnet, die sich nach vorn oder hinter umstülpen lässt. Sie dient zum Einhüllen der Schneidspitze der Nadel beim Zurückziehen derselben.

Die US 2010/0152609 A1 offenbart ein chirurgisches Instrument mit einem Greifer, dem eine sackartige flexible Hülle zugeordnet ist. Diese lässt sich mittels eines gesonderten Schiebers über den Greifer stülpen und zu ziehen, um vom Greifer ergriffenes Gewebe einzuhüllen und ohne Berührung mit anderem Gewebe aus dem behandelten Körper entnehmen zu können.

Die US 2005/0197595 A1 offenbart ein Biopsieinstrument mit einem Container, der einen zusammengefalteten Schlauch enthält. Beim Vorschieben des Biopsieinstruments, zum Beispiel in Brustgewebe, wird das Biopsieinstrument durch den Container geschoben, wobei der dort enthaltene gefaltete Strumpf über das Instrument gezogen und mit in das Gewebe genommen wird. Beim Zurückziehen zieht sich das Instrument in dem Strumpf zurück, sodass Gewebekontamination vermieden wird.

Die US 2007/0123798 A1 beschreibt ein Stanzbiopsiesystem mit einem Rohr, in dem eine Biospienadel längsbeweglich gehalten ist. Das Rohr ist außen mit einem flexiblen Strumpf versehen, der beim Einführen des Rohrs in das Gewebe das im Gewebe gebildete Loch innen auskleidet.

Weiter beschreibt die US 2016/0206295 A1 ein Kryiobiopsieinstrument, das ein äußeres Rohr mit einer stirnseitigen Schneide und einer Kryosonde umfasst, die in dem Rohr längsbeweglich angeordnet ist.

In Gebrauch wird das Instrument in Gewebe eingestochen, wonach die Kryosonde aus dem Rohr heraus in das Gewebe eingestochen und gekühlt wird. Das angefrorene Gewebe wird dann an der Stirnkante des Rohrs freigeschnitten und durch das Rohr geborgen.

Bei der flexiblen endoskopischen Gewebeprobeentnahme (z.B. Gewebeprobe aus dem Pankreas) mit Hilfe einer Kryobiopsiesonde durch eine Organwand (z.B. Magenwand) hindurch können Teile der Probe oder die gesamte Probe während des gesamten Bergewegs oder speziell beim Durchziehen durch die Organwand verloren gehen. Dadurch kann es zur Keim- oder Zellverschleppung kommen.

Zur Bergung von Gewebeproben ist es bekannt, einen Bergeschlauch mit einem Bergebereich vorzusehen, wobei die Sonde mit der Gewebeprobe in den Bergebereich eingezogen wird. Die Sonde wird mit dem Bergeschlauch gleichzeitig entfernt. Der Bergeschlauch der Kryopunktionssonde kann jedoch nicht durch die entsprechende Organwand mit hindurchgeführt werden. Denn der Bergeschlauch hat einen zu großen Durchmesser und dadurch ist ein zu hoher Kraftaufwand nötig, um den Bergeschlauch vorzuschieben. Die Gefahr eines Abstreifens der Probe an der Organwand besteht somit weiterhin. Die Gewebeprobe (z.B. Tumorgewebe) wird durch gesundes Gewebe (z.B. Magenwand) hindurchgezogen, bevor diese in den Bergeschlauch eingezogen wird. Dabei können Tumorzellen verschleppt werden. Es kann zum Verlust von Gewebeproben durch Abstreifen an z.B. der Magenwand kommen.

Aus dem Stand der Technik ist außerdem die Stanzbiopsie bekannt, bei welcher mittels spezieller Nadeltypen (z.B. TruCut) ein zylinderförmiges Biopsat gewonnen werden kann. Dieses ist jedoch oft mit Artefakten behaftet und die Biopsatmenge kann für eine Auswertung zu gering sein.

Aus dem Stand der Technik ist zudem die Feinnadel-Biopsie bekannt, bei der eine Injektionsnadel in die Gewebestelle eingeführt wird, aus der eine Probe entnommen werden soll und Zellen durch die Injektionsnadel abgesaugt werden. Größere Biopsate können damit nicht gewonnen werden.

Aufgabe der Erfindung ist ein verbessertes Konzept zur Gewinnung eines Biopsats anzugeben.

Diese Aufgabe wird mit einer Kryobiopsiesonde nach Anspruch 1 gelöst.

Die erfindungsgemäße Kryobiopsiesonde weist ein Bergeelement zum Befestigen an oder benachbart einer Arbeitsspitze eines Biopsieinstruments (im Folgenden auch Instrument genannt) auf. Die Arbeitsspitze ist dazu eingerichtet, das Biopsat festzuhalten. Die Arbeitsspitze kann auch als Kopf oder Sondenkopf bezeichnet werden. Das Biopsat kann beispielsweise außen an der Arbeitsspitze anhaften, insbesondere angefroren sein, und/oder in der Arbeitsspitze aufgenommen sein. Die Arbeitsspitze kann dazu eingerichtet sein, durch eine Öffnung, insbesondere in einer Gewebewand, zu einem Entnahmeort, beispielsweise einer weiteren Gewebewand hinter der Gewebewand geführt zu werden. Beispielsweise kann die Arbeitsspitze dazu eingerichtet sein, durch eine Magenwand geführt zu werden, um in einem dem Magen benachbarten Organ, z.B. der Pankreas, ein Biopsat zu gewinnen. Die Arbeitsspitze ist dazu eingerichtet, durch eine Öffnung, z.B. durch eine Öffnung in einer Wand aus Gewebe, z. B. einer Organwand oder durch eine Öffnung eines Arbeitskanals eines Endoskops oder Bronchoskops zurückgezogen zu werden. Das Bergeelement ist dazu eingerichtet, beim Zurückziehen der Arbeitsspitze über das Biopsat umgestülpt zu werden, um das Biopsat bei dem Zurückziehen der Arbeitsspitze durch die Öffnung, beispielsweise durch die Organwand, zu umgeben, um das Biopsat beim Zurückziehen durch die Öffnung von der Umgebung, insbesondere quer zur Bergerichtung, insbesondere von der Wand um die Öffnung, zu separieren. Insbesondere kann das Biopsat mittels des Bergeelements beim Zurückziehen des Biopsats durch die Öffnung, insbesondere durch die Öffnung in der Gewebewand, von dem Rand der Öffnung oder der Gewebewand oder dem Gewebe separiert, also auf Abstand zu oder getrennt von dem Rand der Öffnung oder der Gewebewand oder dem Gewebe gehalten werden, beispielsweise um das Biopsat nicht zu verunreinigen. Die Bergeeinrichtung dient zusätzlich dazu, das Gewebe um die Öffnung herum von dem Biopsat, welches Keime oder entartete Zellen enthalten kann, zu separieren, also das Biopsat auf Abstand zu oder getrennt von dem Gewebe zu halten, beispielsweise um die Keime oder entartete Zellen nicht zu verschleppen.

Das Bergeelement wird beim Zurückziehen vorzugsweise von einer ersten Stellung (Orientierung) in eine zweite Stellung (Orientierung) umgestülpt, um das Biopsat zu umgeben. Das Bergeelement weist vorzugsweise eine erste Oberfläche auf, welche beim Bergen, wenn das Bergeelement das Biopsat separiert, dem Biopsat zugewandt ist, und eine zweite Oberfläche auf, welche beim Bergen, wenn das Bergeelement das Biopsat separiert, vom Biopsat abgewandt ist. Beim Umstülpen von der ersten Stellung in die zweite Stellung zum Umgeben des Biopsats, wird die erste Oberfläche, welche in der ersten Stellung nach außen gekehrt ist, durch das Umstülpen nach innen gekehrt, um dem Biopsat zugewendet zu werden. Die zweite Oberfläche wird beim Umstülpen entsprechend nach außen gekehrt.

Mittels des Bergeelements kann das Biopsat und/oder das Gewebe um die Öffnung nicht nur vor Keim- oder Zellverschleppung geschützt werden, sondern das Biopsat kann mittels des Bergeelements auch vor einem mechanischen Abstreifen des Biopsats beim Durchziehen durch die Öffnung geschützt werden. Dies ist insbesondere für Ausführungsformen erfindungsgemäßer Biopsieinstrumente von Bedeutung, mit welchen Biopsate gewonnen werden können, die eine Querabmessung, z.B. Durchmesser, aufweisen, die größer als die Querabmessung der Arbeitsspitze und/oder eines mit der Arbeitsspitze geschaffenen Stichkanals in das Zielgewebe und/oder einer mit der Arbeitsspitze geschaffenen Öffnung in einer Organwand ist.

Das erfindungsgemäße Kryobiopsiesonde kann mit einem oder mehreren der folgenden Merkmale weitergebildet sein:

Wenn das Bergeelement über das Biopsat gestülpt ist, um das Biopsat umfänglich zu umgeben, kann das Biopsat durch das Bergeelement einen mechanischen Schutz erfahren, beispielsweise gegen Abstreifen von der Arbeitsspitze. Das Bergeelement kann dazu eingerichtet sein, das Biopsat umfänglich, insbesondere entlang des Umfangs um den Schaft, an welchem die Arbeitsspitze angeordnet ist, entlang eines Umfangs um die Arbeitsspitze und/oder um das Biopsat, geschlossen zu umgeben, um das Biopsat fluidisch von dem Gewebe zu separieren. Wenn das Biopsat von dem Gewebe mittels des Bergeelements quer, insbesondere senkrecht zur Zurückziehrichtung von der Umgebung fluidisch separiert wird, können Biopsat und Gewebe besonders zuverlässig vor gegenseitiger Kontamination geschützt werden. Der Abschnitt des Bergeelements, welcher das Biopsat umgibt, ist vorzugsweise flüssigkeitsdicht, insbesondere aus flüssigkeitsdichtem Material. Der Abschnitt kann beispielsweise aus Kunststoffsein, beispielsweise aus Silikon oder Polytetrafluorethylen bestehen. Wenn das Bergeelement dazu eingerichtet ist, einen flüssigkeitsdichten Schirm oder Mantel für das Biopsat zu bilden, kann das Bergeelement das Biopsats besonders effektiv gegen Kontamination durch Flüssigkeit abschirmen, insbesondere beim Bergen des Biopsats durch eine enge Öffnung in einer Gewebewand, welche Gewebewand um die Öffnung das Biopsat ohne Bergeelement beim Bergen direkt berühren würde.

Das Bergeelement kann dazu eingerichtet sein, im elastisch nicht verformten Zustand quer zur Zurückziehrichtung oder Bergerichtung bzw. quer zur Längserstreckungsrichtung des Schaftes und/oder der Arbeitsspitze das Biopsat mit Abstand zu umgeben. Der Bergeraum, welchen das umgestülpte Bergeelement für das Biopsat schafft, kann in elastisch nicht verformten Zustand quer zur Bergerichtung einen Innendurchmesser aufweisen, welcher größer ist, als der Durchmesser des Biopsats.

In Ausführungsformen kann ein Abschnitt des Bergeelements zum Umgeben des Biopsats derart nachgiebig sein, dass der Abschnitt, wenn der Abschnitt beim Herausziehen der Arbeitsspitze aus dem Körper des Patienten gegen das Biopsat gedrückt wird, sich an die Form des Biopsats anpasst und an dem Biopsat angeschmiegt bleibt, wenn die Arbeitsspitze aus dem Körper herausgezogen ist.

Das Verhältnis des Durchmessers des Schaftes - an einer Stelle an oder in dem Bergelement gemessen - zu dem größten Außendurchmesser des Bergeelements ist vorzugsweise höchstens 1:1 bis wenigstens 1:6, besonders bevorzugt höchstens 1:3 bis wenigstens 1:5. Ein Verhältnis von 1:1 ist möglich, wenn der größte Durchmesser der Arbeitsspitze in deren Bereich zum Anhaften des Biopsats kleiner ist als der Durchmesser des Schaftes.

Die Länge des Abschnitts des Instruments, welcher zur Aufnahme, insbesondere durch Anhaften, eines Biopsats bestimmt ist und entlang welchem sich der Teil des Bergeelements beim Umgeben des Biopsats erstreckt und damit den Abschnitt separiert, entspricht vorzugsweise dem 2- bis 20-fachen, besonders bevorzugt dem 4- bis 10-fachen, des Durchmessers des Schaftes, gemessen an einer Stelle an oder in dem Bergeelement.

Während das umgestülpte Bergeelement das Biopsat vorzugsweise umfänglich (entlang eines Umfangs um das Biopsat herum) derart umschließt, so das keine Flüssigkeit und/oder Aerosol und/oder Gas quer zur Bergerichtung und/oder radial zur Arbeitsspitze an das Biopsat gelangen kann oder von dem Biopsat in die Umgebung gelangen kann, kann das Bergeelement in Richtung entgegen der Bergerichtung, insbesondere in die Richtung, in welche die Arbeitsspitze weist, offen sein.

Das Bergeelement kann das Biopsat vollständig einschließen, also nicht nur umfänglich umgeben, sondern ein Abschnitt des um das Biopsat umgestülpten Bergelemements kann auch in distale Richtung bzw. in Längserstreckungsrichtung der Arbeitsspitze und/oder des Instruments zwischen dem distalen Ende und der Umgebung angeordnet sein. Wenn das Biopsat von dem umgestülpten Bergeelement vollständig eingehüllt wird, kann eine Keimverschleppung besonders wirksam verhindert werden.

Das Bergeelement kann eine Strukturierung aufweisen, welche dazu eingerichtet ist, beim Zurückziehen der Arbeitsspitze mit der Öffnung, dem Öffnungsrand und/oder der Wand um die Öffnung in Eingriff zu geraten, um das Umstülpen des Bergeelements um das Biopsat zu unterstützen. Die Strukturierung gerät beim Zurückziehen mit der Wand, dem Öffnungsrand und/oder der Öffnungswand in Eingriff und kann dort auf Grund von Formschluss oder Reibschluss vorübergehend hängen bleiben, so dass das Umstülpen des Bergeelements durch das Zurückziehen gefördert wird, bis der Abschnitt des Bergeelement mit der Strukturierung durch die Öffnung gezogen wird. Die Strukturierung kann beispielsweise in einer rauen Oberfläche bestehen oder die Oberflächeninnenstrukturierung kann wenigstens ein Strukturelement oder mehrere Strukturelemente wie Schuppen und/oder Noppen aufweisen. Eine zum Fördern des Umstülpens bestimmte Strukturierung kann beispielsweise an einer Seite eines Teils des Bergeelements ausgebildet sein, welche beim Zurückziehen des Bergeelements beim Umstülpen nach außen gekehrt wird, und/oder eine zum Fördern des Umstülpens bestimmte Strukturierung kann beispielsweise an einer Seite eines Teils des Bergeelements ausgebildet sein, welche beim Zurückziehen des Bergeelements beim Umstülpen nach innen gekehrt wird.

Ausführungsformen der Biopsatbergeeinrichtung können beispielsweise Bergeelemente aufweisen, welche ein Abstützelement aufweisen. Dieses ist bevorzugt quer zur Zurückziehrichtung elastisch verformbar und dazu eingerichtet, sich beim Zurückziehen an der Wand an der Öffnung, bevorzugt um die Öffnung herum, abzustützen, um das Umstülpen zu fördern. Das Abstützelement wird beim Durchführen durch die Öffnung in Richtung zu der Stelle, an der das Biopsat entnommen werden soll, elastisch verformt und nach dem Durchtritt durch die Öffnung, welche durch das Durchführen des Instruments, insbesondere des Bergeelements gedehnt werden kann, nach Wegfall der äußeren Kraft außerhalb der Öffnung bestrebt ist, wieder einen größeren Durchmesser quer zur Öffnungsrichtung bzw. zur Bergerichtung (Zurückziehrichtung) anzunehmen. Das Abstützelement, das auf Grund elastischer Rückstellkräfte wieder eine größeren Durchmesser angenommen hat, vorzugsweise einen größeren Durchmesser als die Öffnung, stützt sich bei dem Zurückziehen an der Wand, welche die Öffnung enthält, ab, bleibt also dort vorübergehend hängen, um die Umstülpung zu fördern. Bevorzugt stützt sich das Abstützelement beim Zurückziehen um die Öffnung herum ab.

Das Abstützelement stützt sich beim Zurückziehen zumindest vorzugsweise solange ab, bis das Bergeelement umgestülpt ist.

Das Abstützelement kann beispielsweise ein elastisch verformbarer Ring sein. Das Abstützelement ist bevorzugt an dem Ende des Bergeelement angeordnet, welches beim Durchtreten durch die Öffnung in die Richtung um ein Biopsat mit der Arbeitsspitze aufzunehmen die Öffnung als letztes verlässt.

Das Bergeelement kann aus einem Material bestehen oder eine Beschichtung aufweisen, welches Material oder Beschichtung dazu eingerichtet ist, um ein Anhaften und/oder Anfrieren des Bergeelements an dem Instrument, beispielsweise einer Kryobiopsiesonde, insbesondere an deren Arbeitsspitze und/oder deren Schaft, und/oder um ein Anfrieren und/oder Anhaften des Biopsats an dem Bergeelement zu verhindern. Beispielsweise kann das Bergeelement aus einem Material bestehen oder eine Beschichtung aufweisen, welches Material oder Beschichtung dazu eingerichtet ist, um ein Anhaften und/oder Anfrieren des Bergeelements an einem Biopsieinstrument zum Gewinnen eines Biopsats, welches Biopsieinstrument mit einer erfindungsgemäßen Biopsatbergeeinrichtung ausgestattet ist, insbesondere ein Anhaften und/oder Anfrieren an dessen Arbeitsspitze und/oder dessen Schaft, zu verhindern.

Wenn das Bergelement bei einer Kryobiobsiesonde verwendet wird, ist zusätzlich oder alternativ das Bergeelement bevorzugt aus einem Material gebildet, welches in seiner Elastizität nicht von der Kühlung der Arbeitsspitze und/oder des Schaftes beeinflusst wird. Insbesondere ist die Elastizität des Teils des Bergeelements, welcher umgestülpt wird, bevorzugt in den Temperaturbereichen temperaturunabhängig, in welchen der umstülpbare Teil bei der Gewinnung des Biopsats mittels einer Kryobiopsiesonde zumindest in Abschnitten des Teils eine Temperatur annehmen kann.

Das Bergeelement kann insbesondere angrenzend oder benachbart zu einer Befestigungsstelle an dem Biopsieinstrument oder benachbart zu einem Befestigungsabschnitt des Bergeelements, welcher an dem Biopsieinstrument fixiert ist, einen Unterabschnitt mit sich entlang eines Längsabschnitts des Biopsieinstruments erweiterndem Außendurchmesser aufweisen. Der Unterabschnitt kann zwischen dem Unterabschnitt und dem Biosieinstrument im umgestülpten Zustand einen Bereich eines Bergeraumes begrenzen. Der Außendurchmesser des Unterabschnitts kann sich zum Durchführen des Biopsieinstruments durch eine Öffnung in einer Wand in einer Richtung erweitern, die der Richtung (Durchführrichtung), in welche das Biopsieinstrument zum Durchführen bewegt wird, entgegengesetzt ist bzw. sich in Durchführrichtung verjüngt, um das Durchführen durch die Öffnung zu vereinfachen. Bevorzugt verjüngt sich der Außendurchmesser des Unterabschnitts bei in der ersten Stellung angeordnetem Bergeelement in Bewegungsrichtung des Instruments zu der Stelle, an welcher das Biopsat entnommen werden soll, und/oder verjüngt sich der Außendurchmesser des Unterabschnitts bei in die zweite Stellung umgestülptem Bergeelement in die Bergerichtung, d.h. in die Richtung, in welche das Biopsieinstrument zum Entfernen der Arbeitsspitze mit dem Biopsat von der Stelle entfernt wird, an welcher das Biopsat entnommen wurde. Auf diese Weise kann das Durchführen des Bergeelements durch die Öffnung vereinfacht sein. Der Unterabschnitt kann sich beispielsweise konisch verjüngen. Der Unterabschnitt ist vorzugsweise elastisch in radialer Richtung bzw. quer zur Bergerichtung verformbar. Damit kann das Durchführen des Bergeelements durch die Öffnung in Durchstechrichtung und/oder das Zurückziehen des Bergeelements durch die Öffnung besonders vereinfacht sein.

Der Unterabschnitt, welche den Abschnitt zum Umgeben des Biopsats mit dem Befestigungsabschnitt verbindet, kann am Übergang zu dem Abschnitt des Bergeelements zum Umgeben des Biopsats einen Außendurchmesser aufweisen, welcher zu dem Außendurchmesser am Übergang zu dem Befestigungsabschnitt in einem Verhältnis von höchstens 1:1 bis mindestens 1:6; vorzugsweise höchstens 1:3 bis mindestens 1:5 steht, wenn das Bergeelement quer zur Längserstreckungsrichtung des Schaftes nicht elastisch verformt ist. Die Angabe des Verhältnisses kann sich auf eine Stellung beziehen, in welcher das freie Ende des Bergeelements in proximale Richtung zeigt, und/oder auf eine Stellung, in welcher das freie Ende des Bergeelements in distale Richtung zeigt.

Bevorzugt weist das Material oder weisen die Materialien des Bergeelements eine Härte von mindestens 20 bis höchstens 80 Shore A, vorzugsweise mindestens 40 bis höchstens 60 Shore A auf. In Ausführungsbeispielen kann das Bergeelement aus Materialien zusammengesetzt sein, welche unterschiedliche Härten gemessen beispielsweise in Shore A aufweisen. Das Bergelement kann beispielsweise in dem Befestigungsabschnitt des Bergeelements ein härteres Material aufweisen als in dem hin zu dem freien Ende des Bergelements angrenzenden Abschnitt (Verbindungsabschnitt) des Bergeelements, welcher den Abschnitt des Bergeelements zum Umgeben des Biopsats mit dem Befestigungsabschnitt verbindet. Alternativ oder zusätzlich kann das Material des Verbindungsabschnitts härter sein als das Material des Abschnitts des Bergelements zum Umgeben des Biopsats sein. Der Härteunterschied zwischen den Materialien kann beispielsweise wenigstens 5 Shore A betragen. In Ausführungsbeispielen kann beispielsweise die Härte der Abschnitte linear, progressiv oder degressiv von dem Abschnitt zum Umgeben als am wenigsten harten Abschnitt, über den Verbindungsabschnitt zu dem Befestigungsabschnitt als am härtesten der drei Abschnitte steigen.

Das Bergeelement kann eine Aufspannstruktur aufweisen, welche sich von einem Basisabschnitt oder -element weg erstreckenden, voneinander in Umfangsrichtung beabstandete, längliche Elemente und/oder Abschnitte aufweist, welche auf Grund der Steifigkeit der Verbindung des Basisabschnitts- oder -elements mit den sich von diesen Weg erstreckenden Elementen und/oder Abschnitt bestrebt sind, bei Wegfall einer äußeren Kraft ihre ursprüngliche Orientierung in Bezug auf den Basisabschnitt - oder element wieder anzunehmen und so die Aufspannstruktur aufzuspannen, nachdem beispielsweise das Basiselement durch eine enge Öffnung in einen weiteren Bereich bewegt worden ist. Die voneinander in Umfangsrichtung beabstandeten länglichen Elemente oder Abschnitte sorgen dafür, dass sich der Unterabschnitt radial kontrolliert zusammendrücken lässt, wenn der Unterabschnitt durch eine Öffnung in einer Gewebewand geschoben wird, und die Verbindung mit dem Basiselement oder abschnitt dafür, dass sich der Unterabschnitt auf Grund der elastischen Rückstellkräfte besonders zuverlässig wieder öffnet, um seine ursprüngliche Form wieder anzunehmen. Das Bergeelement kann länglichen Abschnitte beispielsweise in Form einer entlang des Umfangs abwechselnd zu- und abnehmenden Wanddicke und/oder längliche verstärkende Elementabschnitten aufweisen, welche aus einem anderen Material bestehen können, als der Unterabschnitt, z.B. aus Metall oder einem anderen Polymer.

Wenn das Bergeelement in beispielhaften Ausführungsformen in Längsrichtung aneinander gereihte, unterschiedliche Materialien aufweist, welche Abschnitte des Bergeelements bilden, können Abschnitte unterschiedlicher radialer Steifigkeit gebildet werden. In Ausführungsbeispielen kann ein Verbindungsabschnitt beispielsweise aus einem anderen Werkstoff bestehen als ein Abschnitt zum Umgeben des Biopsats. Damit können Ausführungsbeispiele des erfindungsgemäßen Bergeelements bereitgestellt werden, in welchen der Verbindungsabschnitt eine andere, z.B. größere, radiale Steifigkeit aufweisen kann als der Abschnitt zum Umgeben des Biopsats.

Laut Erfindung ist die größte Wanddicke des Abschnitts des Bergeelements zum Umgeben des Biopsats kleiner als die größte Wanddicke des Verbindungsabschnitts des Bergeelements, welcher den Abschnitt zum Umgeben des Bergeelements mit dem Befestigungsabschnitt verbindet. In Aus-führungsformen mit einem Verbindungsabschnitt, dessen Wanddicke entlang des Umfangs abwechselnd zu- und abnimmt, z.B. um eine Aufspannstruktur zu bilden, kann die Wanddicke zum Vergleich mit der Wanddicke des Abschnitts zum Umgeben des Biopsats an einer Stelle dickster Wanddicke gemessen werden oder an einer Stelle geringster Wanddicke. Mittels über Abschnitte des Bergeelements in Richtung zu dem freien Ende des Bergeelements sich stetig verändernder Wanddicke, insbesondere stetig und vorzugsweise monoton (z.B. linear) in Richtung zu dem freien Ende abnehmender Wanddicke können Abschnitte mit in Richtung freiem Ende abnehmender Steifigkeit gegen radiale Verformung geschaffen sein, was die Positionierbarkeit und die Fähigkeit des Bergeelements unterstützt, zuverlässig über das Biopsat umgestülpt zu werden.

Die Wandung des Bergeelements weist vorzugsweise eine Dicke von einschließlich 0,005 mm bis 2 mm auf. Die Wandung weist vorzugsweise eine Dicke < 0,5 mm auf. Die Durchführung durch eine Organwand, insbesondere eine Magenwand, wird dadurch erleichtert.

Wenn das Bergelement beim Vorschieben des Instruments zu dem Ort, an welchem das Biopsat entnommen werden soll, durch die Öffnung bewegt wird, kann das Bergelement radial zusammengedrückt werden und kann beim Durchtreten durch die Öffnung in die Länge gezogen und insbesondere gedehnt werden, weil hintere Abschnitte des Bergeelements langsamer durch die Öffnung treten als vordere Abschnitte, welche die Öffnung schon passiert haben, durch die Vorschubbewegung des Instruments bewegt werden. Bevorzugt wird das Bergeelement dabei höchstens auf die fünffache ungedehnte Länge, besonders bevorzugt auf höchstens die 1,5fache ungedehnte Länge des Bergeelements, elastisch gedehnt. Die Länge wird vorzugsweise in Längserstreckungsrichtung des Biopsieelements und/oder der Arbeitsspitze gemessen. Wenn damit ein übermäßiges in die Länge Ziehen des Bergeelements beim Durchziehen oder Schieben des Bergeelements durch etwa die Magenwand verhindert wird, sind auch Gewebebiopsien unmittelbar hinter der Magenwand gewinnbar, ohne auf das Umgeben des Biopsats durch das Bergeelement beim Zurückziehen durch die Öffnung verzichten zu müssen.

Das Bergeelement weist bevorzugt eine für Zellen und/oder Gewebe nicht anhaftende erste Oberfläche und/oder zweite Oberfläche auf. Die erste Oberfläche und/oder die zweite Oberfläche kann ihre nicht haftende (in der Haftung stark gehemmte) Eigenschaft beispielsweise auf Grund einer Mikrostrukturierung und/oder einer Plasmabehandlung aufweisen. Alternativ oder zusätzlich weist die Oberfläche des Bergeelements, beispielsweise die erste Oberfläche und/oder die zweite Oberfläche, eine Antihaftbeschichtung gegen das Anhaften von Zellen und/oder Gewebe auf. Die Oberfläche kann beispielsweise mit Fluorpolymer beschichtet sein. Die Oberfläche kann, beispielsweise auf Grund der Beschichtung und/oder -bearbeitung beispielsweise einen Kontaktwinkel von größer oder gleich 90°, besonders bevorzugt größer oder gleich 110° aufweisen. Damit kann verhindert werden, dass durch das Bergeelement Zellen verschleppt werden.

Ausführungsformen erfindungsgemäßer Biopsieinstrumente können insbesondere Kryobiopsiesonden sein. Wenn Abschnitte der Arbeitsspitze der Kryobiopsiesonde abgekühlt werden, kann daran Gewebe festfrieren. Durch Abreißen kann ein gefrorenes Biopsat gewonnen werden. Die tiefen Temperaturen der Arbeitsspitze können beispielsweise durch Ausnutzen des Joule-Thomson-Effekts, wie in dem oben zitierten Stand der Technik angegeben, erreicht werden.

Während das Biopsieinstrument bevorzugt eine Kryobiopsiesonde ist, kann die erfindungsgemäße Biopsatbergeeinrichtung jedoch auch bei anderen Biopsiesonden verwendet werden, um ein Biopsat bei der Entnahme von der Umgebung zu separieren, um die Umgebung und/oder das Biopsat zu schützen und/oder ein Verlorengehen des Biopsats zu verhindern. Beispielsweise kann die Biopsatbergeeinrichtung auch an einer Biosiesonde eingesetzt werden, mit welcher ein Biopsat aus dem Gewebe ausgestanzt wird.

Die Arbeitsspitze ist vorzugsweise dazu eingerichtet, so dass mit der Arbeitsspitze eine Organwand durchstochen werden kann, die Öffnung in der Organwand also mittels der Arbeitsspitze geschaffen werden kann.

Das Bergelement kann beispielsweise durch Umspritzen einer Arbeitsspitze und/oder eine Schaftes eines Biopsieinstruments, insbesondere einer Kryobiopsiesonde, hergestellt und mit dem Biopsieinstrument dabei verbunden werden.

Bevorzugt werden Instrumente mit wenigstens einem Bergelementen, welches austauschbar ist, um das Bergelement, insbesondere nach der ersten Benutzung, entsorgen und das Instrument mit einem anderen Bergeelement benutzen zu können.

Für das Positionieren des Biopsieinstruments zur Trennung der Probe aus dem Gewebe kann der Abschnitt des Bergeelements zum Umgeben des Biopsats in einen Abschnitt des Bergeelements aufnehmbar, insbesondere einssteckbar, einrollbar und/oder einfaltbar sein, welcher Abschnitt den Abschnitt zum Umgeben des Biopsats mit dem Biopsieinstrument verbindet. In einem Zustand erfindungsgemäßer Ausführungsformen des Instruments ist der Abschnitt des Bergeelements zum Umgeben des Biopsats in einen Abschnitt des Bergeelements aufgenommen, insbesondere eingesteckt, eingerollt und/oder eingefaltet, welcher Abschnitt den Abschnitt zum Umgeben des Biopsats mit dem Biopsieinstrument verbindet.

Das Biopsieinstrument kann ein Sicherungselement aufweisen, welches dazu eingerichtet ist, ein ungewolltes Umstülpen während der Positionierung der Arbeitsspitze zum Abtrennen der Probe zu verhindern. Für das Zurückziehen des Biosieinstruments zur Entnahme der Probe kann das Sicherungselement außer Funktion gesetzt werden, so dass ein Umstülpen beim Zurückziehen möglich ist.

Weitere vorteilhafte Merkmale, mit denen die erfindungsgemäße Bergeeinrichtung oder das erfindungsgemäß Biopsieinstrument weiter gebildet sein können oder bevorzugte Ausführungsformen der erfindungsgemäßen Bergeeinrich-tung oder des erfindungsgemäßen Biopsieinstruments ergeben sich aus nachfolgender Beschreibung und den Figuren. Es zeigen schematisch und beispielhaft:
Figur la ein Ausführungsbeispiel eines erfindungsgemäßen Biopsieinstruments in einer abschnittsweisen Ansicht mit einer beispielhaften erfindungsgemäßen Bergeeinrichtung in einer ersten Orientierung,
Figur 1b das Ausführungsbeispiel des erfindungsgemäßen Biopsieinstruments gemäß Figur la in einer abschnittsweisen Ansicht mit der Bergeeinrichtung in einer zweiten Orientierung,
Figuren 2a bis 2i ein Ausführungsbeispiel eines erfindungsgemäßen Biopsieinstruments beispielsweise des Instruments gemäß Figur 1, bei der Entnahme eines Biopsats,
Figur 2i' eine Abwandlung des Ausführungsbeispiels eines erfindungsgemäßen Biopsieinstruments gemäß Figuren 2a bis 2i,
Figuren 3a und 3b - ein Ausführungsbeispiel gemäß einer alternativen Ausführungsform eines erfindungsgemäßen Bergeelements,
Figuren 3c und 3d - eine Abwandlung des Ausführungsbeispiels gemäß Figuren 3a und 3b,
Figuren 4-12 - weitere Ausführungsbeispiele erfindungsgemäßer Biopsieinstrumente bzw. Bergeelemente.

Bei dem erfindungsgemäßen Instrument handelt es sich um eine Kryobiopsiesonde. Bei einem solchen ist die Arbeitsspitze des Instruments abkühlbar, beispielsweise durch Ausnutzen des Joule-Thomson-Effekts im Inneren der Arbeitsspitze, wie dies im Stand der Technik hinlänglich bekannt ist. Die Arbeitsspitze wird auf das Gewebe an der Stelle, an der das Biopsat zu entnehmen ist, gedrückt oder in das Gewebe eingedrückt oder gestochen. Durch das starke Abkühlen der Arbeitsspitze kann das der Arbeitsspitze benachbarte Gewebe an der Arbeitsspitze festgefroren werden. Durch Entfernen der Arbeitsspitze von dem Ort, an dem das Gewebe an der Arbeitsspitze festgefroren ist, insbesondere durch eine ruckartige Bewegung, kann das gefrorene Gewebe aus dem Gewebeverbund herausgerissen und geborgen werden, um es zu untersuchen.

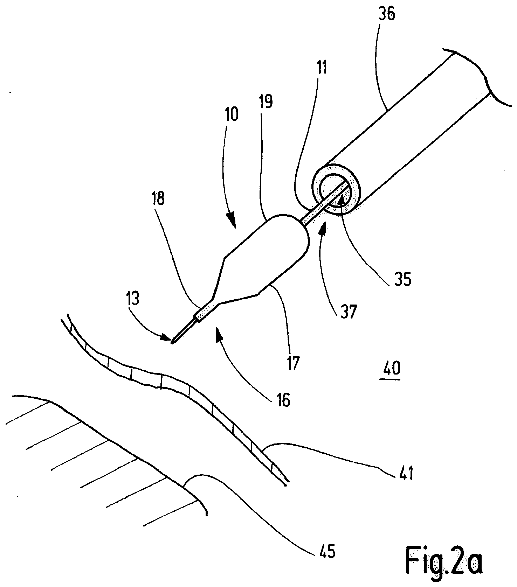

Ausführungsbeispiele erfindungsgemäße Biopsieinstrumente 10, wie eines beispielsweise in Figur la dargestellt ist, weisen vorzugsweise einen länglichen flexiblen Schaft 11 auf, an dessen distalem Ende 12 eine Arbeitsspitze 13 angeordnet ist. Wie dargestellt, kann der Schaft 11 von wärmeisolierenden Kunststoff 14 ummantelt sein, während die Arbeitsspitze 13 vorzugsweise außen aus blankem Metall besteht, um einen guten Wärmeübergang von dem Gewebe auf die Arbeitsspitze 13 zu erhalten. Die Arbeitsspitze 13 ist vorzugsweise dazu eingerichtet, mit der Arbeitsspitze 13 eine Gewebe- oder Organwand, beispielsweise die Magenwand 41 (s. beispielsweise Figur 2a), zu durchstechen. Das Biopsieinstrument 10 ist mit einem Ausführungsbeispiel einer erfindungsgemäßen Biopsatbergeeinrichtung 16 ausgerüstet. Benachbart zu dem distalen Ende 15 des Instruments 10, insbesondere benachbart zu der Arbeitsspitze 13, ist ein Bergeelement 17 um den Schaft 11 und/oder die Arbeitsspitze 13 angeordnet, welches Bergeelement 17 beispielsweise aus Kunststoff besteht, beispielsweise aus PTFE oder Silikon. Das Bergeelement 17 weist einen Befestigungsabschnitt 18 auf, über welchen das Bergeelement 17 an dem Schaft 11 benachbart zu der Arbeitsspitze 13 und/oder an der Arbeitsspitze 13 befestigt ist. Der Befestigungsabschnitt 18 ist vorzugsweise relativ zu dem Schaft 11 und/oder der Arbeitsspitze 13 längs des Schaftes 11 und/oder der Arbeitsspitze 13 nicht verschiebbar an dem Schaft 11 und/oder der Arbeitsspitze 13 fixiert. An den Befestigungsabschnitt 18 grenzt ein Teil 19 des Bergeelements 17 zum Umgeben des Biopsats 20 an, welches an der Arbeitsspitze 13 festgefroren werden kann, um das Biopsat 20 zum Bergen festzuhalten.

Der Teil 19 zum Umgeben des Biopsats 20 in Figur la in einer ersten Stellung bzw. Orientierung gezeigt, in welcher eine erste Oberfläche 25 (der Außenseite in dieser Orientierung) des Teils 19 nach außen orientiert ist, und eine zweite Oberfläche 26 (der Innenseite in dieser Orientierung) nach innen orientiert ist. Das offene, freie Ende 27 des Teils 19 zum Umgeben des Biopsats 20 ist in dieser Stellung in eine Richtung orientiert, die der Richtung in welche die Arbeitsspitze 13 zeigt, entgegengesetzt ist.

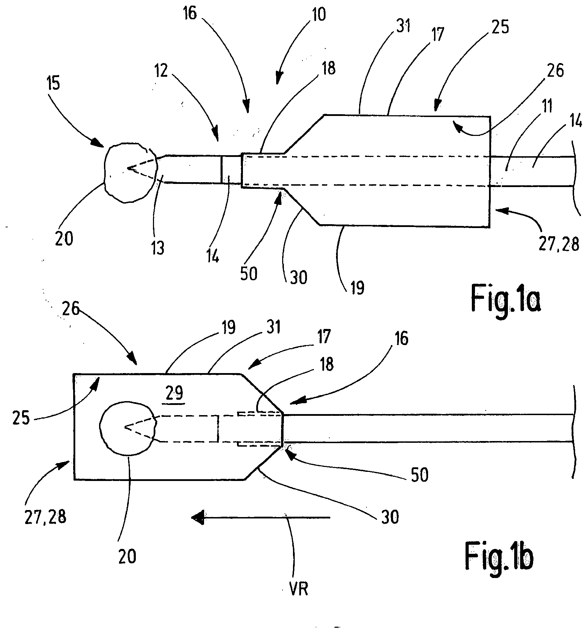

Der Teil 19 zum Umgeben des Biopsats 20 ist von dieser Stellung bzw. Orientierung in eine zweite Stellung bzw. Orientierung umstülpbar. Dabei wird das offene, freie Ende 27 des Bergeelements 17 um den Schaft 11 und die Arbeitsspitze 13 über den Befestigungsabschnitt 18 hinaus in Richtung Arbeitsspitze 13 bzw. distales Ende 15 des Instruments 10 bewegt. Der Befestigungsabschnitt 18 bleibt dabei bevorzugt unbeweglich relativ zum Schaft 11 und/oder der Arbeitsspitze 13 am Schaft 11 und/oder der Arbeitsspitze 13 fixiert. Bei der Bewegung des Endes 27 und Umstülpen des Teils 19 des Bergeelements 17 wird die zweite Oberfläche 26, welche nicht umgestülpt nach Innen wies, zumindest bereichsweise nach außen orientiert, und wird die erste Oberfläche 25, welche nicht ungestülpt nach Außen weist, zumindest bereichsweise nach innen orientiert, um dem Schaft 11 und/oder der Arbeitsspitze 13 und/oder dem Biopsat 20 zugewandt zu werden. Das Instrument 10 mit Bergeelement 17 in umgestülpter Orientierung zeigt Figur 1b. Das freie Ende 27 des Bergeelements 17, so wie umgestülpt, bzw. die Öffnung 28 am freien Ende 27 des Teils 19 zum Umgeben des Biopsats 20, so wie umgestülpt, ist in dieser Stellung in die Richtung orientiert, in welche die Arbeitsspitze 13 weist. Der Teil 19 des Bergeelements 17 zum Umgeben des Biopsats 20 ist so lang, dass das Ende 27 des umgestülpten Bergeelements 17 bis über das Biopsat 20 reicht. Das zum umfänglichen Umgeben des Biopsats 20 umgestülpte Bergeelement 17 umgibt einen Bergeraum 29, welcher bei nicht elastisch verformtem Bergeelement 17 bevorzugt einen größeren Innendurchmesser D1 aufweist als das Biopsat 20. Bevorzugt umgibt das umgestülpte Bergeelement 17 das Biopsat 20 um das Biopsat 20 herum mit radialem Abstand zwischen Berge-element und Biopsat 20.

Der an den Befestigungsabschnitt 18 angrenzende Unterabschnitt 30 des Teils 19 der Bergelements 17 zum Umgeben des Biopsats 20 kann radial (quer zum Schaft 11 und/oder zur Arbeitsspitze 13 und/oder zum Biopsat 20) elastisch verformbar sein. Der Unterabschnitt 30 kann ke-gel- oder konusförmig sein. Ein daran angrenzender weiterer Unterabschnitt 31 zum Umgeben des Biopsats 20 bei umgestülptem Bergeelement 17, welcher zwischen dem freien Ende 27 und dem Unterabschnitt 30 angeordnet ist, kann verformbar, aber nicht elastisch verformbar sein. Während der elastisch verformbare Unterabschnitt bestrebt ist, bei wegfall einer verformenden Kraft in seine ursprüngliche Form zurückzukehren, kann dies auf den nicht-elastisch verformbaren weiteren Unterabschnitt 31 nicht oder nur in geringerem Maße zutreffen. Der an den elastisch verformbaren Unterabschnitt 30 angrenzende weitere Unterabschnitt 31 kann jedoch auch elastisch verformbar sein.

Figur 2a zeigt ein Ausführungsbeispiel eines erfindungsgemäßen Instruments 10, beispielsweise das erfindungsgemäße Instrument 10 wie im Zusammenhang mit Figur la und 1b beschrieben, dessen Schaft 11 durch einen Arbeitskanal 35 eines Endoskops 36 geführt ist und dessen Arbeitsspitze 13 und Bergeelement 17 vor dem distalen Ende 37 des Endoskops 36 angeordnet ist. Das Endoskop 36 ist durch die Speiseröhre in den Magen 40 des Patienten eingeführt und mit der Arbeitsspitze 13 vor der Magenwand 41 des Patienten angeordnet. Ziel ist es, mit dem Instrument 10 ein Biopsat 20 aus der Bauchspeicheldrüse 45 des Patienten zu gewinnen, welche benachbart zu dem Magen 40 ist.

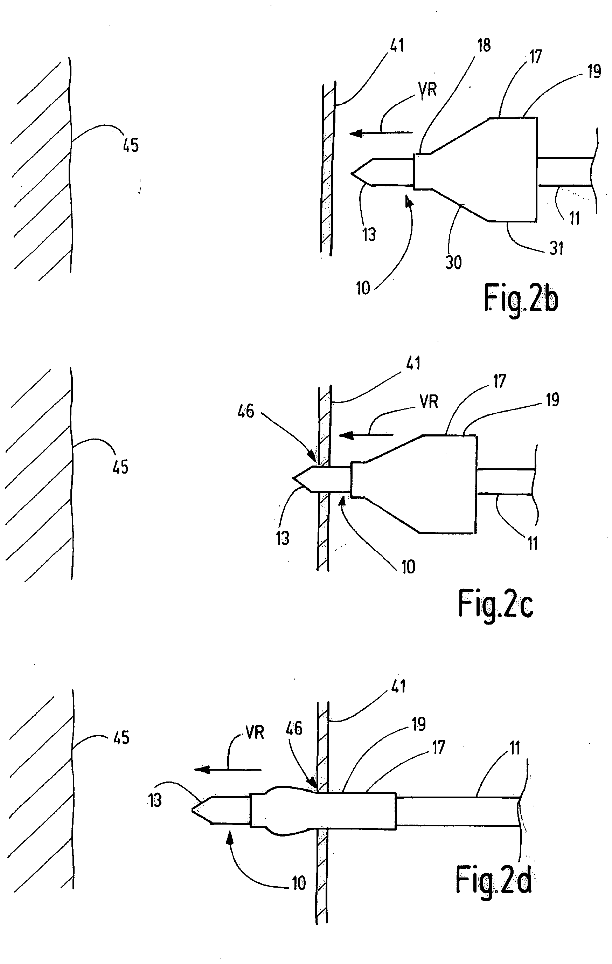

Dazu wird, wie in Figur 2b veranschaulicht, das Instrument 10 in Durchstech- oder Vorschieberichtung VR in Richtung Magenwand 41 vorgeschoben und mittels der Arbeitsspitze wird die Magenwand 41 durchstochen, also eine Öffnung 46 in der Magenwand 41 geschaffen, wie es in Figur 2c veranschaulicht ist.

Das Instrument 10 wird weiter durch die so geschaffene Öffnung 46 in der Magenwand 41 geschoben, wobei auch der Teil 19 des Bergeelements 17 zum Umgeben des Biopsats 20 durch die Öffnung 46 geschoben wird. Beim Durchtreten durch die Öffnung 46 wird das Bergeelement 17 radial zusammengequetscht. Die Wandung des Bergeelements 17, insbesondere die Wandung des Teils 19 zum Umgeben des Biopsats 20, weist vorzugsweise eine Dicke von einschließlich 0,005 mm bis einschließlich 2 mm auf. Die Wandung, insbesondere die Wandung des Teils 19 zum Umgeben des Biopsats 20, weist vorzugsweise eine Dicke < 0,5 mm auf. Insbesondere wenn die maximale Dicke der Wandung des Bergeelements 17 oder zumindest die maximale Dicke des Teils 19, der das Biopsat 20 umgibt, zwischen einschließlich 0,005 mm bis einschließlich 2 mm liegt oder kleiner als 0,5 mm ist, wird die Durchführung durch eine Organwand, wie beispielsgemäß die Magenwand 41, dadurch erleichtert.

Wenn, wie auch in der Ausführungsform gemäß Figur la, 1b, ein an den Befestigungsabschnitt 18 angrenzender Unterabschnitt 30 des Teils 19 zum Umgeben des Biopsats 20 sich in Vorschieberichtung VR verjüngend, insbesondere konisch verjüngen ausgebildet ist, kann das Durchschieben des Bergeelements 17 durch die Öffnung 46 in der Magenwand 41 erleichtert sein.

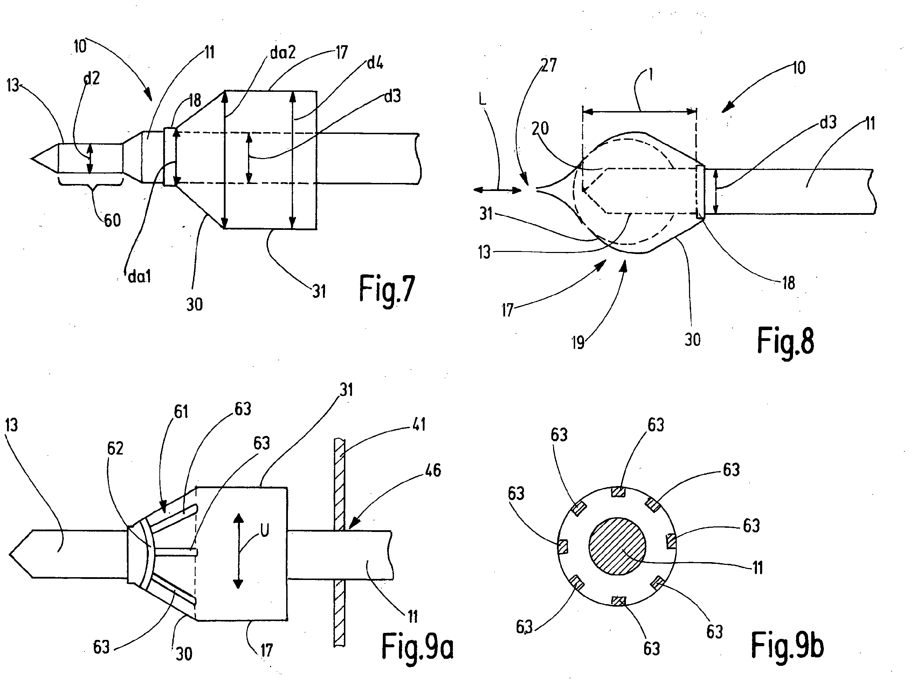

Der Unterabschnitt 30, welche den Abschnitt 31 zum Umgeben des Biopsats 20 mit dem Befestigungsabschnitt 18 verbindet, kann am Übergang zu dem Abschnitt 31 des Ber-geelements 17 zum Umgeben des Biopsats 20 einen Außendurchmesser dal (s. beispielhaft Figur 7) aufweisen, welcher zu dem Außendurchmesser da2 am Übergang zu dem Befestigungsabschnitt 18 in einem Verhältnis von höchstens 1:1 bis mindestens 1:6; vorzugsweise höchstens 1:3 bis mindestens 1:5 steht, wenn das Bergeelement 17 quer zur Längserstreckungs-richtung des Schaftes 11 nicht elastisch verformt ist. Die Angabe des Verhältnisses kann sich auf eine Stellung beziehen, in welcher das freie Ende 27 des Bergeelements 17 in proximale Richtung zeigt, und/oder auf eine Stellung, in welcher das freie Ende 27 des Bergeelements 17 in distale Richtung zeigt.

Der Unterabschnitt 30 ist vorzugsweise nicht direkt mit dem Schaft 11 oder der Arbeitsspitze 13, sondern über den Befestigungsabschnitt 18 mit dem Instrument 10 verbunden. Der Unterabschnitt 30 ist radial bzw. quer zur Zurückziehrichtung ZR und/oder zur Durchstechrichtung VR bevorzugt elastisch verformbar. Wenn der Unterabschnitt 30 zwischen Magenwand und Bauchspeicheldrüse aus der Öffnung 46 herausgetreten ist, kann dieser aufgrund seiner Elastizität wieder seine ursprüngliche Form annehmen.

Wenn das Bergelement 17 beim Vorschieben des Instruments 10 zu dem Ort an der Bauchspeicheldrüse 45, an welchem das Biopsat 20 entnommen werden soll, durch die Öffnung 46 bewegt wird, kann das Bergelement 17 beim Durchgezogen bzw. Durchgeschoben werden durch die Öffnung 46 im Zuge des Durchstechens der Magenwand 41 in die Länge gezogen und insbesondere gedehnt werden, weil hintere Abschnitte des Teils 19 des Bergeelements 17 langsamer durch die Öffnung 46 treten als vordere Abschnitte vorbewegt werden, welche schon durch die Öffnung 46 getreten sind. Bevorzugt wird das Bergeelement 17 und/oder der Teil 19 des Bergeelements, der an den Befestigungsabschnitt 18 angrenzt, höchstens auf die fünffache ungedehnte Länge, besonders bevorzugt auf höchstens die 1,5fache ungedehnte Länge des Berge-elements 17 bzw. des Teils 19, elastisch gedehnt. Die Länge wird vorzugsweise in Längserstreckungsrichtung des Biop-sieinstruments 10 und/oder der Arbeitsspitze 13 gemessen. Wenn damit ein übermäßiges in die Länge Ziehen des Berge-elements 17 beim Durchstechen durch etwa die Magenwand 41 verhindert wird, sind auch Gewebebiopsien unmittelbar hinter der Magenwand 41 gewinnbar, ohne auf das Umgeben des Biopsats 20 durch das Bergeelement 17 beim Zurückziehen durch die Öffnung 46 verzichten zu müssen.

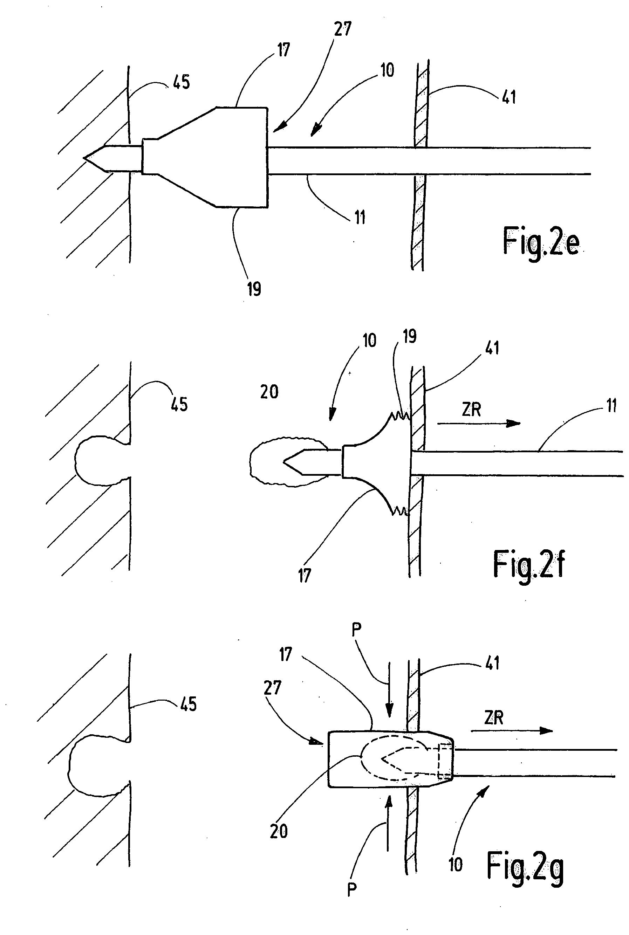

Das Instrument 10 wird weiter durch die Öffnung 46 geschoben und wenn das Bergelement 17 die Öffnung 46 zwischen Magenwand 41 und Bauchspeicheldrüse 45 verlassen hat, können elastische Abschnitte 30, sofern vorhanden, des Bergeelements 17 die ursprüngliche Form durch radiale Ausdehnung wieder annehmen. Das Instrument 10 wird weiter vorgeschoben, bis die Arbeitsspitze 13 an das Gewebe der Pankreas 45 drückt oder in das Gewebe der Pankreas 45 eingestochen ist (Figur 2e).

Die Arbeitsspitze 13 wird gekühlt und dadurch wird Gewebe an der Arbeitsspitze 13 festgefroren. Das Gewebe wird durch ruckartiges Zurückziehen des Instruments 10 in Bergerichtung ZR (Pfeil in Figur 2f) aus dem Pankreas 45 gelöst.

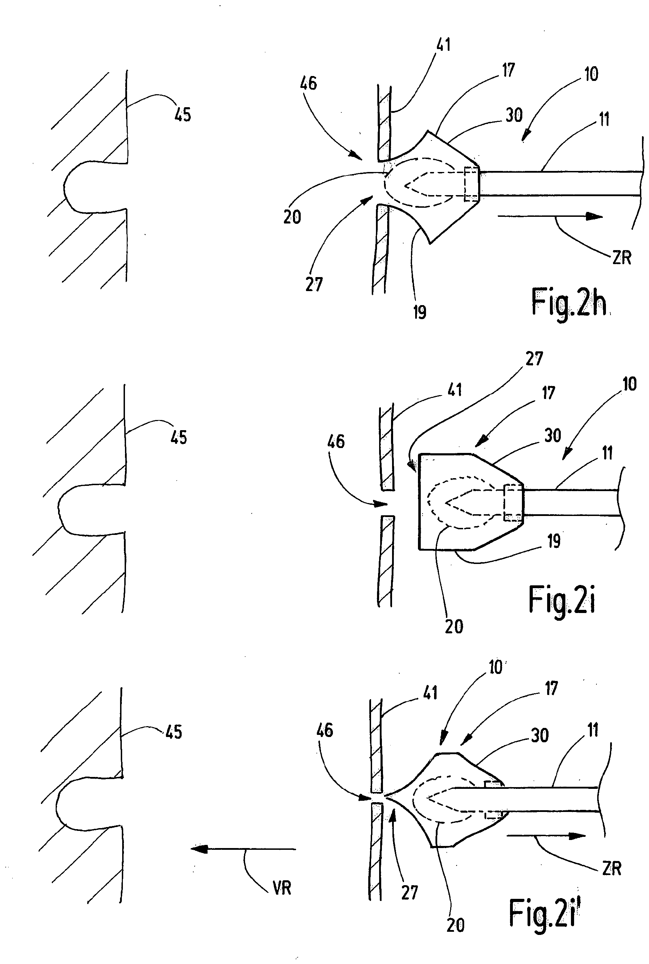

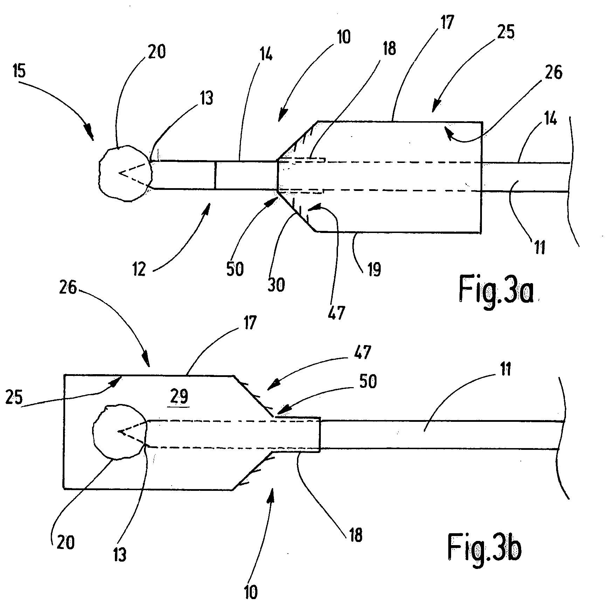

Die Arbeitsspitze 13 wird durch die Öffnung 46 aus dem Zwischenraum zwischen Pankreas 45 und Magenwand 41 herausgezogen. Dabei gelangt das Bergeelement 17 zuvor in Anlage und/oder Eingriff mit der Magenwand 41 und wird durch die Zurückziehbewegung des Schaftes 11 und die Anlage an der Magenwand 41 bzw. den Eingriff mit der Magenwand 41 über die Arbeitsspitze 13 bzw. über das Biopsat 20 gestülpt, um dieses wie durch die Pfeile P in Figur 2g veranschaulicht ist, quer zur Bergerichtung ZR abzuschirmen, insbesondere beim Durchziehen durch die Magenwand 41 (siehe insbesondere Figur 2h). Das Bergeelement 17 kann eine Strukturierung 47 (s. beispielhaft Figur 3b) aufweisen, welche dazu eingerichtet ist, beim Zurückziehen der Arbeitsspitze 13 mit dem Rand der Öffnung 46 oder der Magenwand 41 um die Öffnung 46 in Eingriff zu geraten, um das Umstülpen des Bergeelements 17 um das Biopsat 20 zu unterstützen. Die Strukturierung kann beispielsweise in einer rauen Oberfläche bestehen und/oder die Oberflächeninnen-strukturierung kann Schuppen und/oder Noppen aufweisen, so dass die Strukturierung beim Zurückziehen mit der Magenwand 41 bzw. dem Rand der Öffnung 46 in Eingriff gerät und dort hängen bleibt, so dass das Bergeelement 17 durch das Zurückziehen umgestülpt wird. Das Durchziehen des Bergeelements 17 durch die Öffnung 46 in der Magenwand 41 unter Umstülpen des Teils 19 des Bergeelements 17 zum Umgeben des Biopsats 20 kann erleichtert sein, wenn der ungestülpte, an den Befestigungsabschnitt 18 angrenzende Unterabschnitt 30 des Teils 19 zum Umgeben des Biopsats 20 sich außen in Bergerichtung ZR (Zurückziehrichtung) verjüngend ausgebildet ist, wenn der Außendurchmesser in Berge- und Zurückzieh-richtung ZR beispielsweise konisch abnimmt.

Das Bergeelement 17 kann dazu eingerichtet sein, das Biopsat 20 umfänglich, entlang eines Umfangs um die Arbeitsspitze 13 geschlossen zu umgeben, um das Biopsat 20 radial fluidisch von dem Gewebe, insbesondere dem Magen-wandgewebe, zu separieren. Wenn das Biopsat 20 von dem Gewebe mittels des Bergeelements 17 quer, insbesondere senkrecht zur Zurückziehrichtung (RZ) von der Umgebung flui-disch separiert wird, können Biopsat 20 und Gewebe besonders zuverlässig vor gegenseitiger Kontamination geschützt werden. Der Abschnitt des Bergeelements, welcher das Biop-sat umgibt, ist entsprechend vorzugsweise flüssigkeitsdicht, insbesondere aus flüssigkeitsdichtem Material. Wenn das Bergelement 17 quer zur Zurückziehrichtung ZR einen flüssigkeitsdichten Mantel oder Schirm bildet, kann dieses das Biopsat 20, insbesondere beim Durchziehen durch die Öffnung 46 (Figuren 2g, 2h ), quer zur Längsachse des Instruments 10 gegen Kontamination durch Flüssigkeiten abschirmen. Während das umgestülpte Bergeelement das Biopsat vorzugsweise umfänglich derart umschließt, so das keine Flüssigkeit und/oder Aerosol und/oder Gas quer zur Berge-richtung und/oder radial zur Arbeitsspitze und/oder zum Schaft an das Biopsat gelangen kann oder von dem Biopsat in die Umgebung gelangen kann, ist das Bergeelement in Richtung entgegen der Bergerichtung, insbesondere in die Richtung, in welche die Arbeitsspitze weist, in Ausführungsbeispielen offen.

In Figur 2h ist veranschaulicht, wie das Bergeelement beim Durchziehen durch die Öffnung zusammengequetscht wird. Das über das Biopsat gestülpte Bergeelement unterstützt hierbei, dass das Biopsat beim Durchziehen durch die Wand nicht an dem Öffnungsrand hängen bleibt, so dass das Biopsat beim Durchziehen durch die Öffnung in der Organwand nicht von der Arbeitsspitze abgestreift wird. Das Bergeelement besteht vorzugsweise aus einem Material oder weist vorzugsweise eine Beschichtung auf, welches Material oder Beschichtung dazu eingerichtet ist, ein Anhaften und/oder Anfrieren des Bergeelements an der Kryobiopsiesonde, insbesondere an deren Arbeitsspitze, und/oder um ein Anfrieren und/oder Anhaften des Biopsats an dem Bergeelement zu verhindern. Das Bergeelement kann beispielsweise aus Kunststoff sein, beispielsweise PTFE oder Silikon. Bevorzugt weist die Oberfläche des Bergeelements (die erste Oberfläche und/oder die zweite Oberfläche) eine Antihaftbeschichtung gegen das Anhaften von Zellen und/oder Gewebe auf.

Figur 2i zeigt, dass das Bergeelement auch nach dem Durchziehen durch die Magenwand, nachdem das Bergeelement und die Arbeitsspitze die Öffnung verlassen haben, umgestülpt ist und das Biopsat in radialer Richtung abschirmt. Insbesondere wenn das Bergeelement das Biopsat umfänglich geschlossen umgibt, ist das Biopsat weder mit Magenwandzellen oder Keimen von der Magenwand verunreinigt, noch hat das Biopsat die Mageninnenwand beim Durchziehen durch die Öffnung berührt, so dass Zellen, insbesondere entartete Zellen, oder Keime von dem Biopsat in der Magenwand hinterlassen wären.

Das umgestülpte Bergeelement 17 kann das Biopsat 20 auch vor ungewolltem Abstreifen an dem distalen Ende 37 des Endoskops 36 oder in dem Arbeitskanal 35 des Endoskops 36 schützen, wenn die Arbeitsspitze 13 mit dem Biopsat 20 in den Arbeitskanal 35 des Endoskops 36 zurück hineingezogen wird, beispielsweise um das Biopsat 20 durch den Arbeitskanal 35 zu bergen.

Das Bergeelement kann das Biopsat vollständig einschließen, also nicht nur umfänglich umgeben, sondern das umgestülpte Bergeelement, ggf. zusammen mit dem Instrument, kann das Biopsat nach allen Seiten einschließen. Ins-besondere kann ein Bereich des Teils des umgestülpten Ber-geelements zum Umgeben des Biopsats auch distal zwischen dem distalen Ende der Arbeitsspitze und der Umgebung angeordnet sein. Dazu ist das Bergeelement vorzugsweise so lang, dass das umgestülpte Bergeelement bis über das dista-le Ende der Arbeitsspitze und/oder bis über das distale Ende des Biopsats reicht. Beim Zurückziehen des Bergeelements kann insbesondere der über die Arbeitsspitze und/oder das Biopsat in Richtung distal überstehende Unterabschnitt des Teils des Bergeelements zum Umgeben des Biopsats zusammengequetscht werden und gegenüberliegende Abschnitte der ersten Oberfläche können in Anlage gebracht werden und durch Adhesionskräfte zusammenhalten. Dies ist beispielhaft in Figur 2i³ gezeigt: Das freie Ende 27 des Bergelements 17 kann dazu eingerichtet sein, beim Durchgezogenwerden durch die Öffnung 46 von dem elastisch verformten Gewebe der Magenwand 41, welches die Öffnung 46 umgibt, zusammengeknäult zu werden und durch Adhäsionskräfte zusammenzuhalten, um das Ende 27 weitgehend zu verschließen. Das Biopsat 20 kann so vollständig eingehüllt werden, also auch in Richtung distal (in Vorschieberichtung VR) mittels des Bergeelements 17 von der Umgebung separiert werden. Wenn das Biopsat 20 von dem umgestülpten Bergeelement 17 vollständig eingehüllt wird, kann eine Keimverschleppung besonders wirksam verhindert werden.

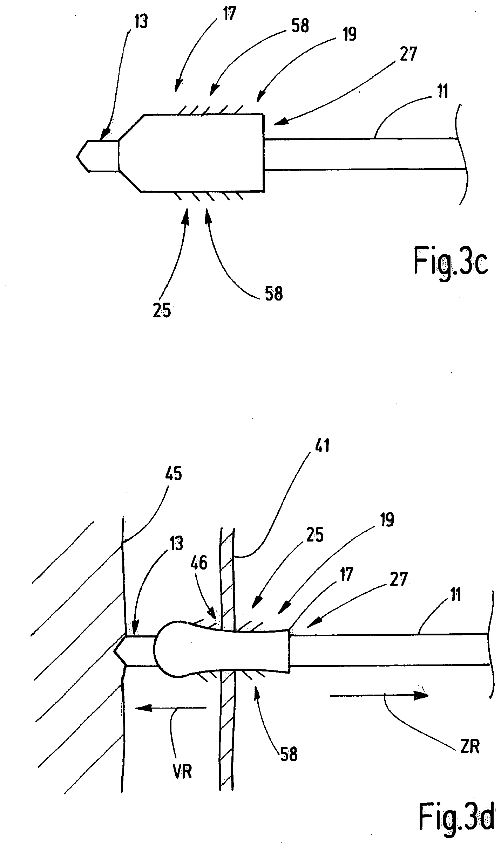

Der Befestigungsabschnitt 18 kann an den Schaft des Instruments 10 beispielsweise geklebt oder geklemmt sein. Das Bergeelement 17 ist vorzugsweise fluiddicht an dem Instrument 10 befestigt. An den Befestigungsabschnitt 18 schließt sich an einer Anschlussstelle 50 der Teil 19 des Bergeelements 17 an, welcher umgestülpt wird und welcher das Biopsat 20 radial abschirmt. In dem Ausführungsbeispiel gemäß Figur 1 ist die Anschlussstelle 50 proximal an dem Befestigungsabschnitt 18 angeordnet. In der Ausführungsform gemäß Figur 3 ist die Anschlussstelle 50 distal an dem Befestigungsabschnitt 18 angeordnet. Die gemäß Figur 3 veränderte Fixierung des Bergeelements 17 kann die Umstülpeigenschaften des Bergeelements 17 verbessern, da der Teil des Bergeelements 17 zum Abschirmen des Biopsats 20 beim Umstülpen nicht über den Befestigungsabschnitt 18 gezogen werden muss. Figur 3b zeigt die Montageposition bzw. die mittels Durchziehen durch die Öffnung 46 in der Organwand 417 umgestülpte Orientierung des Bergeelements. Das Bergeelement kann eine Trichterform aufweisen, wobei der Trichterhals den Befestigungsabschnitt 18 bilden kann. Zur Montage kann das Bergeelement 17 auf den Schaft 11 gezogen oder geschoben werden, wobei der Schaft 11 durch den Trich-terhals tritt. Figur 3a zeigt die Orientierung zum Einstechen in die Organwand 41 (s. z.B. Figuren 2a bis 2i), um zu der Stelle zu gelangen, an welcher das Biopsat entnommen werden soll. Das Bergeelement 17 kann zunächst in der Montageposition bzw. Orientierung (Figur 3b) montiert werden und dann in die Orientierung zum Durchstechen wie in Figur 3a gezeigt umgestülpt werden. Beim Zurückziehen des Berge-elements durch die Öffnung 46 nach dem Aufnehmen der Gewebeprobe wird das Bergeelement 17 in die distale Richtung VR über die Arbeitsspitze 13 zurück in die Orientierung umgestülpt, welche in Figur 3b veranschaulicht ist. Das Bergeelement 17 ist (dies gilt beispielsweise auch für das Bergeelement 17 gemäß Figur la, lb) ist sowohl in distale Richtung VR als auch in proximale Richtung ZR umstülpbar.

Figur 3c zeigt eine Abbildung einer Abwandlung der Ausführungsform gemäß Figuren 3a, b wie sie auch in den übrigen beschriebenen Ausführungsformen vorhanden sein kann. Der Abwandlung gemäß Figur 3c entsprechend weist der Teil 19 des Bergelements 17 zum Umgeben des zu entnehmenden Biopsats an seiner ersten Oberfläche 25, welche zunächst nach außen orientiert ist und welche beim Bergen des Biopsats durch Umstülpen des Bergeelements 17 nach innen gekehrt wird, eine Strukturierung 58 auf. Das Instrument 10 ist in Figur 3d in einer Position, welche Position einerseits der Position des Instruments 10 dargestellt in Figur 2d entspricht, da sich die Arbeitsspitze 13 zwischen Magenwand 41 und Pankreas 45 befindet und der Teil 19 des Berge-elements zum Umgeben eines Biopsats in der Öffnung 46 angeordnet ist. Andererseits ist die Arbeitsspitze 13 an das Gewebe des Pankreas 45 gedrückt oder, wie es auch Figur 2e zeigt, in das Gewebe des Pankreas 45 eingestochen. Wenn das Instrument 10 aus der beschriebenen und in Figur 3d dargestellten Position zurückgezogen wird, ohne das Instrument 10 zuvor derart weiter in Vorschubrichtung VR schieben zu können, dass der Teil 19 des Bergeelements 17 zwischen die Magenwand 41 und das Pankreas 45 gelangt, gerät die Strukturierung 58 mit der Magenwand spätestens beim Zurückziehen in form- und/oder reibschlüssigen Eingriff. Dadurch wird ein Umstülpen des Bergelements 17 beim Zurückziehen in Bergerichtung ZR gefördert. Die Strukturierung 58 kann beispielsweise durch eine raue Oberfläche gebildet sein und/oder die Strukturierung 58 kann durch wenigstens ein oder mehrere Strukturelemente, wie beispielsweise Schuppen, welche Widerhaken bilden können, und/oder Noppen gebildet sein. Die Strukturierung 58 bietet insbesondere dann einen Vorteil, wenn sich das Zielgebiet, aus welchem das Biopsat gewonnen werden soll, derart nah hinter der Magenwand 41 oder einer anderen Organwand befindet, so dass die Arbeitsspitze 13 sich bereits in oder an dem Gewebe des Pankreas 45 oder eines anderen Organs befindet, während der Teil 19 des Bergelements 17 zum Umgeben in einer Figur 3d entsprechenden Position ist, also noch nicht vollständig durch die Öffnung 46 gelangt ist. Denn in einem solchen Fall ist es möglich, dass das Ende 27 des Bergelements 17 nicht zwischen Magenwand 41 und Pankreas 45 gelangt und das Ende 27 des Bergeelements 17 deshalb beim Zurückziehen nicht in Eingriff mit der Magenwand 41 oder in Anlage an die Magenwandfläche ist, welche dem Pankreas 45 zugewandt ist.

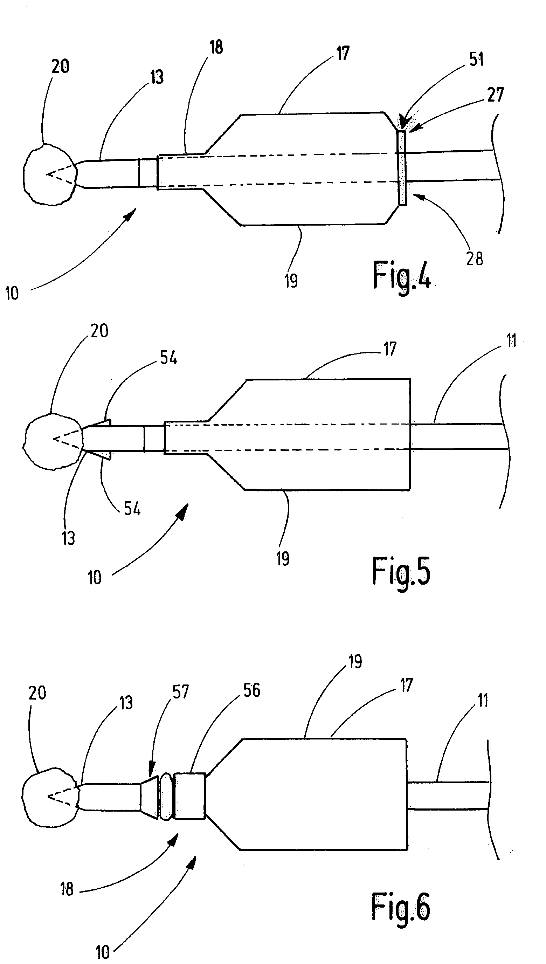

Figur 4 zeigt ein weiteres Ausführungsbeispiel eines erfindungsgemäßen Instruments 10. An dem freien Ende des Umstülpabschnitts des Bergeelements ist ein elastischer, beispielsweise kreisrunder oder polygoner, Ring 51 ausgebildet, welcher ein Abstützelement bildet. Der Ring 51 weist einen größeren Innendurchmesser als die Öffnung 46 in der Organwand 41 (s. u.a. Figuren 2a bis 2i, welche das Schaffen der Öffnung 46 in der Organwand 41 und das Bergen des Biopsats 20 durch die Öffnung 46 zeigen) auf. Beim Durchschieben des Instruments 10 durch die Öffnung 46, um ein Biopsat 20 zu gewinnen, wird der Ring 51 zusammengequetscht, um durch die Öffnung 46 zu passen. Wenn der Ring 51 die Öffnung 46 auf der anderen Seite der Organwand 41 verlassen hat, entspannt sich der Ring 51 aufgrund seiner Elastizität wieder, ist also aufgrund der Elastizität bestrebt, nach Wegfall der äußeren Kraft außerhalb der Öffnung 46 wieder einen größeren Durchmesser quer zur Vor-schieberichtung VR und/oder seine ursprüngliche Form anzunehmen. Beim Zurückziehen stützt sich das Bergeelement 17 mittels des Rings 51 um die Öffnung 46 radial von der Öffnung 46 beabstandet an der Organwand 41 ab und fördert somit das Umstülpen des Bergeelements 17. Das Abstützelement 51 ist bevorzugt derart an dem Ende 27 des Bergeelement 17 angeordnet, so dass das Abstützelement 51 beim Zurückziehen des Instruments 10 durch die Öffnung 46 mit gewonnenem Biopsat 20 erst durch die Öffnung 46 in der Organwand 41 tritt, nachdem das Biopsat 20, abgeschirmt mittels des Bergeelement 17 in die Öffnung 46 eingetreten ist oder die Öffnung 46 in der Organwand 41 verlassen hat. Das Abstütze-lement 51 ist bevorzugt dazu eingerichtet, sich beim Zurückziehen zumindest vorzugsweise solange an der Organwand 46 abzustützen, bis das Bergeelement 17 umgestülpt ist.

Wie in Figur 4 veranschaulicht, kann der Durchmesser der Öffnung 28 am Ende 27 des Bergelements 17, welche Öffnung 28 wie im dargestellten Ausführungsbeispiel von dem Ring 51 gebildet sein kann, vorzugsweise kleiner sein als der Innendurchmesser des Bergeraums 53.

Figur 5 zeigt eine Ausführungsform des erfindungsgemäßen Instruments 10, welches an der Arbeitsspitze 13 angeordnete Schneidelemente 54 aufweist, welche die Durchführbarkeit durch die Organwand zu der Stelle, an welcher das Biopsat20 entnommen werden soll, verbessern.

Figur 6 zeigt ein weiteres Ausführungsbeispiel eines erfindungsgemäßen Instruments 10, bei welchem der Befestigungsabschnitt 18 mittels eines Befestigungsrings 56 umschlossen und auf dem Schaft 11 festgeklemmt (gekrimpt) ist, um den Übergang von dem Schaftdurchmesser bzw. dem Durchmesser der Arbeitsspitze an den Durchmesser des Befestigungsabschnitts 18 bzw. des Befestigungsrings 56 anzupassen, ist zwischen der Arbeitsspitze 13 und dem Befestigungsabschnitt 18 ein Formelement 57 mit sich in Richtung zu dem Befestigungsabschnitt 18 erweiterndem Außendurchmesser, beispielsweise sich konisch erweiterndem Außendurchmesser, angeordnet. Dieses Formelement 57 erleichtert das Durchführen des Instruments 10 durch die Öffnung in der Organwand, zumindest in Durchstechrichtung.

Das Biopsieinstrument ist eine Kryobiopsiesonde.

Während das Bergeelement im Wesentlichen als einstückiges Teil oder flexible Hülle ausgebildet ist, insbesondere als nahtlos einstückiges Teil, kann das Bergeelement auch aus mehreren Teilen bestehen, welche miteinander zueinander beweglich zu einem mechanischen Element verbunden sind. Beispielsweise kann das Bergeelement ähnlich wie ein Faltenbalg aufgebaut sein. Insbesondere in Ausführungsformen, in denen das Bergeelement als einstückiges Teil ausgebildet ist, kann durch die maßliche Ausgestaltung des Bergeelements und/oder unterschiedliche Werkstoffe für unterschiedliche Abschnitte des Bergeelements die Steifigkeit der unterschiedlichen Abschnitte des Bergelements gezielt beeinflusst werden.

Figur 7 zeigt ein Ausführungsbeispiel eines Biopsieinstruments 10 mit einer Arbeitsspitze 13 mit einem Bereich 60, der zum Anhaften des Biopsats bestimmt ist. Der Bereich 60 weist einen größten Durchmesser d2 auf, welcher kleiner ist als der Durchmesser d3 des Schaftes 11 gemessen an einer Stelle an oder in dem Bergelement 17. Dies macht ein Ausführungsbeispiel möglich, in welchem das Verhältnis des Durchmessers d3 des Schaftes 11 - an einer Stelle an oder in dem Bergelement 17 gemessen - zu dem größten Außendurchmesser d4 des Bergeelements 1:1 ist. In dem dargestellten Ausführungsbeispiel ist das Verhältnis größer als 1:1. Das Verhältnis ist bevorzugt höchstens 1:1 bis wenigstens 1:6, besonders bevorzugt höchstens 1:3 bis wenigstens 1:5.

Figur 8 zeigt anhand eines weiteren Ausführungsbeispiels, dass die Länge 1 des Abschnitts des Instruments 10, welcher zur Aufnahme, insbesondere durch Anhaften, eines Biopsats 20 bestimmt ist und entlang welchem sich der Teil 19 des Bergeelements 17 beim Umgeben des Biopsats 20 erstreckt und damit den Abschnitt separiert, vorzugsweise dem 2 bis 20-fachen, besonders bevorzugt dem 4 bis 10-fachen, des Durchmessers d3 des Schaftes 11 gemessen an einer Stelle an oder in dem Bergeelement 17 entspricht. Figur 8 veranschaulicht ein Bergeelement, welches ein Biopsat umhüllt, außerhalb des Körpers des Patienten, wo keine Kräfte auf das Bergeelement von außen wirken, welche das Bergeelement zusammendrücken würden. Der Abschnitt 31 des Bergeelements 17 zum Umgeben des Biopsats 20 kann derart nachgiebig sein, dass der Abschnitt 31, wenn der Abschnitt 31 beim Herausziehen der Arbeitsspitze 13 aus dem Körper des Patienten gegen das Biopsat 20 gedrückt wird, sich an das Biop-sat 20 anpasst und an dem Biopsat 20 angeschmiegt bleibt, wenn die Arbeitsspitze 13 aus dem Körper herausgezogen ist und somit die äußere Kraft auf Grund des Widerstands des Gewebes des Patienten gegen Verformung weggefallen ist, welche Kraft den Abschnitt 31 beim Herausziehen gegen das Biopsat 20 drückt.

Das Material oder die Materialien des Bergeelements können in Ausführungsbeispielen Härten von mindestens 20 bis höchstens 80 Shore A, vorzugsweise mindestens 40 bis höchstens 60 Shore A aufweisen. In Ausführungsbeispielen kann das Bergeelement aus Materialien, z.B. unterschiedlichen Silikonen, zusammengesetzt sein, welche unterschiedliche Härten aufweisen. Die Härte kann in Längsrichtung L des Bergeelement in Richtung zu dem freien Ende 27 des Bergeelements 17 abnehmen, z.B. im Verbindungsabschnitt 30 größer sein als im Abschnitt 31 zum Umgeben des Biopsats 20. Figur 8 zeigt ein Ausführungsbeispiel eines Bergelements, welches in dem Befestigungsabschnitt des Bergeelements beispielsweise ein härteres Material aufweisen kann als in dem hin zu dem freien Ende 27 des Bergelements 17 angrenzenden Verbindungsabschnitt 30 des Bergeelements 17, welcher den Abschnitt 31 des Bergeelements 17 zum Umgeben des Biopsats 20 mit dem Befestigungsabschnitt 18 verbindet. Alternativ oder zusätzlich kann das Material des Verbindungsabschnitts 30 härter sein als das Material des Abschnitts 31 des Bergelements 17 zum Umgeben des Biopsats 20 sein. Der Härteunterschied zwischen den Materialien kann beispielsweise wenigstens 5 Shore A betragen. In Ausführungsbeispielen kann beispielsweise die Härte der Abschnitte linear, progressiv oder degressiv von dem Abschnitt 31 zum Umgeben als am wenigsten harten Abschnitt, über den Verbindungsabschnitt 30 zu dem Befestigungsabschnitt 18 als am härtesten der drei Abschnitte 18, 30, 31 steigen.

Figur 9 veranschaulicht anhand eines Ausführungsbeispiels, dass Ausführungsformen des Bergeelements 17 eine Aufspannstruktur 61 aufweisen kann. Das Bergeelement 17 kann eine Aufspannstruktur 61 aufweisen, welche sich von einem Basisabschnitt oder -element 62 weg erstreckenden, voneinander in Umfangsrichtung U beabstandete, längliche Elemente und/oder Abschnitte 63 aufweist, welche auf Grund der Steifigkeit der Verbindungen des Basisabschnitts- oder -elements 62 mit den sich von diesen Weg erstreckenden Elementen und/oder Abschnitten 63 bestrebt sind, bei Wegfall einer äu-ßeren Kraft ihre ursprüngliche Orientierung in Bezug auf den Basisabschnitt - oder element 62 wieder anzunehmen und so die Aufspannstruktur 61 aufzuspannen, nachdem beispielsweise der Basisabschnitt und/oder -element 62 durch eine enge Öffnung 46 einer Gewebewand in einen weiteren Bereich bewegt worden ist. Die voneinander in Umfangsrichtung beabstandeten länglichen Elemente oder -Abschnitte 63 sorgen dafür, dass sich der Unterabschnitt 30 radial kontrolliert zusammendrücken lässt, wenn der Unterabschnitt 30 durch eine Öffnung 46 in einer Gewebewand geschoben wird, und die Verbindung mit dem Basisabschnitt oder -element 62 dafür, dass sich der Unterabschnitt 30 auf Grund der elastischen Rückstellkräfte besonders zuverlässig wieder öffnet, um seine ursprüngliche Form wieder anzunehmen. Figuren 9a und 9b veranschaulichen, dass das Bergeelement 17 längliche Abschnitte 63 beispielsweise in Form einer entlang der Umfangsrichtung abwechselnd zu- und abnehmende Wanddicke ausgebildet sein können und/oder längliche verstärkende Elementabschnitten 63 aufweisen kann, welche aus einem anderen Material bestehen können, als der Unterabschnitt 30, z.B. aus Metall oder einem anderen Polymer. Die Bereiche unterschiedlicher Wanddicke können gemeinsam aus dem gleichen Material urgeformt sein und/oder auf ein Grundmaterial des Bergelements 17 ist in länglichen Bereichen gleiches oder anderes Material als Verdickung aufgetragen. Die länglichen Abschnitte 63 können sich bis in den Abschnitt 31 zum Umgeben des Biopsats erstrecken oder in dem Verbindungsabschnitt 30 vor dem Abschnitt 31 enden.

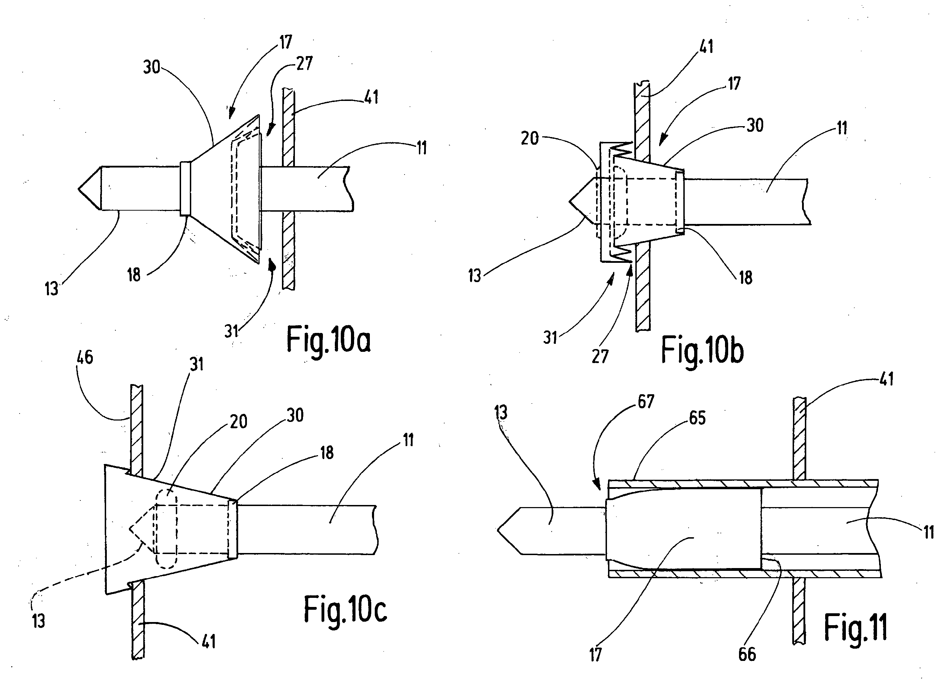

In Ausführungsformen ist der Abschnitt 31 des Bergeelements 17 zum Umgeben des Biopsats 20 für das Einstechen des Biopsieinstruments zu dessen Positionierung in dem Verbindungsabschnitt 30 des Bergeelements 17 aufnehmbar. Bevorzugt ist der Abschnitt 30 zum Umgeben des Biopsats 20 in den Verbindungsabschnitt 30 einroll- und/oder einfaltbar. Figur 10a zeigt ein Beispiel eines Biopsieinstruments 10 nach dem Durchstechen einer Organwand 41 zur Positionierung. Das freie Ende 27 des Bergeelements bzw. die Öffnung in den Verbindungsabschnitt 30 zeigt von dem distalen Ende der Arbeitsspitze 13 weg. Der Abschnitt 31 des Bergeelements 17 am freien Ende 27 ist in den Verbindungsabschnitt 30 eingefaltet. Dadurch ist verhindert, dass das Bergeelement 17 beim Einstechen oder Einführen zu weit nach proximal in die Länge gezogen worden ist. Der eingefaltete und/oder eingerollte Bereich bildet einen Ring mit relativ großem Widerstand gegen radiales Verformen, was das Aufspannen nach dem Durchstechen der Wand 41 fördert. Wenn das Biopsieinstrument 10 zurück durch die Wand 41 zogen wird, um die Probe 20 zu entnehmen (Figur 10b), wird das Bergeelement im Wesentlichen bis auf den Befestigungsabschnitt umgestülpt und der Ring, welcher durch Einfalten und/oder Einrollen entstanden ist, wird nach außen gekehrt. Beim Zurückziehen wird der Abschnitt 31 zum Umgeben des Biopsats 20 automatisch entfaltet und dabei über das Biopsat gezogen (Figur 10c).

Erfindungsgemäße Biopsieinstrumente 10 können ein Sicherungselement 65 aufweisen, welches dazu eingerichtet ist, ein ungewolltes Umstülpen des Bergeelements 17 während der Positionierung der Arbeitsspitze 13 zum Abtrennen des Biopsats 20 zu verhindern. Für das Zurückziehen des Biosieinstruments 10 zur Entnahme des Biopsats kann das Sicherungselement 65 außer Funktion gesetzt werden, so dass ein Umstülpen beim Zurückziehen möglich ist. Das Sicherungselement 65 kann das Bergeelement 17 beispielsweise umschließen und so das Umstülpen verhindern. Das Sicherungselement 65 kann von dem Bergeelement 17 abziehbar sein, während die Arbeitsspitze 13 in dem Gewebe positioniert ist, um das Umstülpen frei zu geben. Das Sicherungselement 65 umschließt vorzugsweise einen in proximale Richtung zeigenden Rand 66 des Bergeelements 17, welcher von dem freien Ende 27 oder beispielsweise einem durch Einrollen und/oder Einfalten, wie im Zusammenhang mit dem Ausführungsbeispiel gemäß Figuren 10a bis 10c beschrieben, gebildeten Ring gebildet sein. Denn der Rand 66 ist dazu bestimmt, beim Zurückziehen derart in Eingriff mit der Gewebewand 41 zu gelangen, dass das Bergeelement 17 umgestülpt wird. Das Sicherungselement 65 kann mit dem Biopsieinstrument 10 und/oder dem Bergeelement 17 verbunden sein, wobei die Verbindung 67 gelöst werden muss, um die Sicherung aufzuheben. Die Verbindung kann dazu eingerichtet sein, beim Lösen zerstört werden zu müssen. Die Verbindung kann dazu eingerichtet sein, beim Kontakt mit Flüssigkeit, insbesondere Gewebeflüssigkeit, aufgelöst oder zumindest geschwächt zu werden, so dass das Sicherungselement 65 von dem Bergeelement 17 oder dem Biopsieinstrument 10 gelöst werden kann. Figur 11 zeigt ein Ausführungsbeispiel eines Sicherungselements 65 in Form eines Hüllschlauches. Der Hüllschlauch 65 kann beispielsweise punktuell mit einer mit geringer Kraft zerstörbaren Verbindung 67 mit dem Bergelement 17 verbunden sein oder das Bergeelement 17 lose umgeben. Die Länge des Hüllschlauchs 65 ist vorzugsweise derart bemessen, dass sich der Hüllschlauch 65 bei positionierter Arbeitsspitze 13 wenigstens über den Rand 66 des Bergelements 17 oder sogar wenigstens durch die Öffnung 46 in der Gewebewand 41 erstreckt. Das Sicherungselement 65 ist dazu eingerichtet, dass der Bediener, nach dem Erreichen der Endposition des Biopsieinstruments 10 (Position bei der Biopsat entnommen wird) und vor dem Zurückziehen der Arbeitsspitze 13 mit dem Biopsat, den Hüllschlauch 65 in proximale Richtung von dem Bergeelement 17 zurückziehen kann, ggf. unter Zerstörung einer Verbindung 67 des Sicherungselements 65 mit dem Bergeelement 65 oder einem anderen Abschnitt des Biopsieinstruments 10.

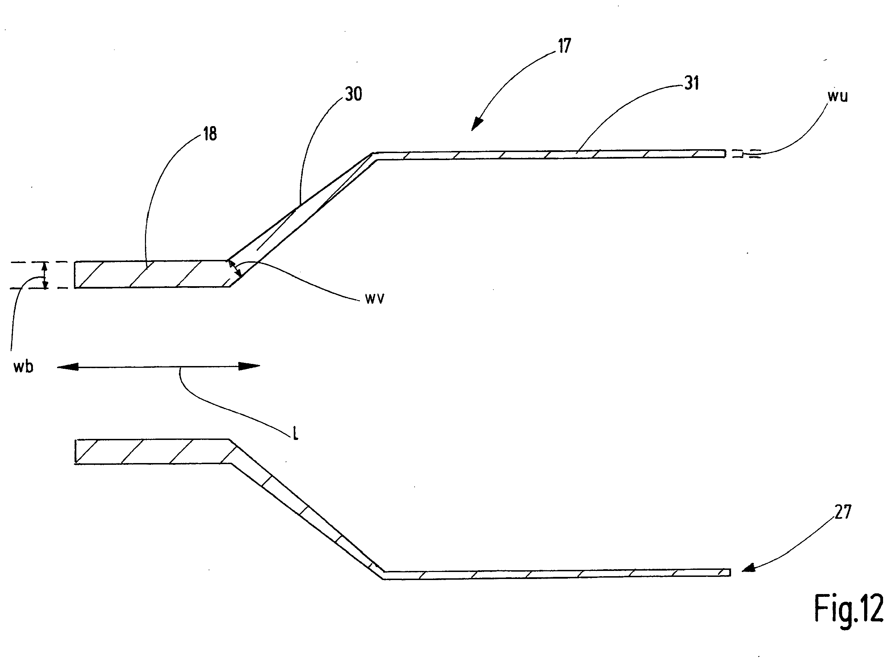

Figur 12 veranschaulicht ein erfindungsgemäßes 17 mit in Richtung zu dem freien Ende 27 des Bergeelements abnehmender Wanddicke. Die größte Wanddicke wv des Verbindungsabschnitts 30 ist größer als die größte Wanddicke wu des Abschnitts 31 zum Umgeben des Biopsats. Die größte Wanddicke wb des Befestigungsabschnitts 18 kann größer sein als die größte Wanddicke wv des Verbindungsabschnitts 30. In Ausführungsformen, welche wie im Zusammenhang mit Figuren 9a, 9b beschrieben, kann die größte Wanddicke wv eines länglichen Abschnitts 63 oder die größte Wanddicke zwischen zwei benachbarten länglichen Abschnitten 63 größer sein als die größte Wanddicke wb des Abschnitts 31 zum Umgeben des Biopsats und/oder kleiner als die größte Wanddicke wb des Befestigungsabschnitts 18. Die größte Wanddicke wu kann, wenn sich die Abschnitte 63 der Aufspannstruktur 61 bis in den Abschnitt 31 zum Umgeben des Biopsats 20 erstrecken, beispielsweise außerhalb der länglichen Abschnitte 63 gemessen werden. Die Wanddicke des Bergelements kann von einem zum, hin zu dem freien Ende 27 angrenzenden, Abschnitt - beispielsweise vom Verbindungsabschnitt 30 zum Abschnitt 31 zum Umgeben - gleitend (stetig) abnehmen. Die Wanddicke kann beispielsweise linear in Richtung zu dem freien Ende 27 abnehmen. Anstelle der größten Wanddicke können die über die Längen der einzelnen Abschnitte gemittelten Wanddicken verglichen werden, wobei die mittlere Wanddicke beispielsweise in Richtung zu dem freien Ende 27 des Bergeelements 17 abnehmen kann.

Merkmale hierin beschriebenen Ausführungsformen können miteinander kombiniert werden. Der Schutzumfang der Erfindung wird jedoch durch die Ansprüche bestimmt. Beispielsweise können Merkmale beschrieben im Zusammenhang mit Figur 12 mit Merkmalen, welche die Härte unterschiedlicher Materialien, die Anpassbarkeit des Abschnitts 31 zum Umgeben des Biopsats 20, die Durchmesserverhältnisse an den Enden des Verbindungsabschnitts 30, einer Aufspannstruktur 61 und/oder die Einroll- oder Einfaltbarkeit des Abschnitts 31 zum Umgeben in den Verbindungsabschnitt 30 betreffen miteinander in Ausführungsformen kombiniert sein.

Merkmale wie unterschiedlicher Wanddicke, Materialien unterschiedlicher Härte und/oder einer Aufspannstruktur 65 sind Beispiele von Merkmalen, mit welchen Ausführungsformen gebildet werden können, welche eine in Richtung zu dem freien Ende 27 abnehmende Steifigkeit des Bergeelements gegen radiale Verformung aufweisen. Beispielsweise kann der Verbindungsabschnitt 30 steifer gegen radiale Verformung sein als der Abschnitt 31 zum Umgeben des Biopsats 20, zumindest wenn dieser ausgerollt und/oder entfaltet ist.

### Bezugszeichenliste:

| | | |
|---|---|---|
| 10 | | Kryobiopsiesonde/Instrument |
| 11 | | Schaft |
| 12 | | distales Ende des Schaftes |
| 13 | | Arbeitsspitze |
| 14 | | Kunststoffmantel |
| 15 | | Distales Ende des Instruments |
| 16 | | Biopsatbergeeinrichtung |
| 17 | | Bergeelement |
| 18 | | Befestigungsabschnitt |
| 19 | | Teil des Bergeelements zum Umgeben des Biopsats |
| 20 | | Biopsat |
| 25 | | Erste Oberfläche |
| 26 | | Zweite Oberfläche |
| 27 | | Ende |
| 28 | | Öffnung |
| 29 | | Bergeraum |
| 30 | | Unterabschnitt/Verbindungsabschnitt |
| 31 | | Weiterer Unterabschnitt/Abschnitt zum Umgeben des Biopsats |
| 35 | | Arbeitskanal |
| 36 | | Endoskop |
| 37 | | Distales Ende des Endoskops |
| 40 | | Magen |
| 41 | | Magenwand |
| 45 | | Bauspeicheldrüse/Pankreas |
| 46 | | Öffnung |
| 47 | | Strukturierung |
| 50 | | Anschlussstelle |
| 51 | | Ring/Abstützelement |
| | | |
| 53 | | Innendurchmesser Bergeraum |
| 54 | | Schneidelemente |
| 56 | | Befestigungsring |
| 57 | | Formelement |
| 58 | | Strukturierung |
| 60 | | Bereich |
| 61 | | Aufspannstruktur |
| 62 | | Basisabschnitt oder -element |
| 63 | | längliches Element oder Abschnitt |
| 65 | | Sicherungselement |
| 66 | | Rand |
| 67 | | Verbindung |
| D1 | | Innendurchmesser Bergeraum |
| VR | | Durchstechrichtung/Vorschieberichtung/distale Richtung |
| ZR | | Zurückziehrichtung/Bergerichtung/proximale Richtung |
| P | | Pfeile |
| da1 | | Außendurchmesser |
| da2 | | Außendurchmesser |
| d2 | | Durchmesser des Bereiches 60 |
| d3 | | Durchmesser des Schaftes 11 |
| d4 | | Außendurchmesser des Bergeelements 17 |
| 1 | | Länge |
| U | | Umfangsrichtung |
| wb | | Wanddicke |
| wv | | Wanddicke |
| Wu | | Wanddicke |
| L | | Längsrichtung |

## Patentansprüche

1. Kryobiopsiesonde mit einer Biopsatbergeeinrichtung (16) zum Bergen eines Biopsats (20) durch eine Öffnung (46) mit einem Bergeelement (17) zum Befestigen an oder benachbart einer Arbeitsspitze (13) der Kryobiopsiesonde, wobei das Bergeelement (17) dazu eingerichtet ist, beim Zurückziehen der Arbeitsspitze (13) durch die Öffnung (46) über das Biopsat (20) umgestülpt zu werden, um das Biopsat (20) bei dem Zurückziehen der Arbeitsspitze (13) durch die Öffnung (46) zu umgeben, um das Biopsat (20) beim Zurückziehen durch die Öffnung (46) von der Umgebung zu separieren, wobei das Bergeelement (17) in Längsrichtung (L) des Bergeelements (17) aneinander gereihte Abschnitte (18, 30, 31) des Bergeelements (17) aufweist, wobei das Bergelement (17) einen Befestigungsabschnitt (18), einen Abschnitt (31) zum Umgeben des Biopsats und einen Verbindungsabschnitt (30) aufweist, welcher den Abschnitt (31) zum Umgeben des Biopsats (20) mit dem Befestigungsabschnitt (18) verbindet, wobei die Wanddicke (wu) des Abschnitts (31) zum Umgeben des Biopsats (20) kleiner ist als die Wanddicke (wv) des Verbindungsabschnitts (30) .

2. Kryobiopsiesonde nach Anspruch 1, wobei das Bergeelement (17) dazu eingerichtet ist, das Biopsat (20) umfänglich geschlossen zu umgeben, um das Biopsat (20) fluidisch von der Umgebung zu separieren.

3. Kryobiopsiesonde nach einem der vorstehenden Ansprüche, wobei das Bergeelement (17) eine Strukturierung aufweist, welche dazu eingerichtet ist, bei dem Zurückziehen der Arbeitsspitze mit der Wand um die Öffnung in Eingriff zu geraten, um das Umstülpen des Bergeelements (17) über das Biopsat beim Zurückbewegen des Biopsieinstruments (10) durch die Öffnung (46) zu unterstützen.

4. Kryobiopsiesonde nach einem der vorstehenden Ansprüche, wobei das Bergeelement (17)ein Abstützelement (51) aufweist, welches quer zur Zurückziehrichtung (ZR) elastisch verformbar ist und dazu eingerichtet ist, sich beim Zurückziehen an einer Wand (41) an der Öffnung (46) abzustützen.

5. Kryobiopsiesonde nach einem der vorstehenden Ansprüche, wobei das Bergeelement (17) aus einem Material besteht oder eine Beschichtung aufweist, welches Material oder Beschichtung dazu eingerichtet oder ausgewählt ist, ein Anfrieren des Bergeelements (17) an dem Biopsieinstrument (10) und/oder ein Anfrieren des Biopsats (20) an dem Bergeelement (17) zu verhindern.

6. Kryobiopsiesonde nach einem der vorstehenden Ansprüche, wobei das Bergeelement (17) angrenzend oder benachbart zu der Befestigungsstelle des Bergeelements (17) an dem Biopsieinstrument (10), einen Abschnitt (30) mit sich entlang eines Längsabschnitts des Biopsieinstruments (10) erweiterndem Außendurchmesser aufweist.

7. Kryobiopsiesonde nach einem der vorstehenden Ansprüche, wobei das Bergeelement (17) dazu eingerichtet ist, dass sich das Bergeelement (17) beim Durchstechen einer Organwand (41) auf höchstens die fünffache Länge, besonders bevorzugt auf höchstens die 1,5-fache Länge, des ungedehnten Bergeelements (17) elastisch dehnt.

8. Kryobiopsiesonde nach einem der vorstehenden Ansprüche, wobei die Wandung des Berge-elements (17) eine Dicke von einschließlich 0,005 mm bis 2 mm, vorzugsweise kleiner 0,5 mm, aufweist.

9. Kryobiopsiesonde nach einem der vorstehenden Ansprüche, wobei sich die Materialien, aus welchen aneinander gereihte Abschnitte bestehen, von Abschnitt zu Abschnitt unterscheiden.

10. Kryobiopsiesonde nach einem der vorstehenden Ansprüche, wobei das Bergeelement (17) eine für Zellen und/oder Gewebe nicht anhaftende Oberfläche aufweist.

11. Kryobiopsiesonde (10) nach einem der vorstehenden Ansprüche 1 mit einer Arbeitsspitze (13), wobei die Arbeitsspitze (13) dazu ausgebildet ist, mit der Arbeitsspitze (13) eine Organwand (41) zu durchstechen.

12. Kryobiopsiesonde (10) nach einem der vorstehenden Ansprüche, wobei der Abschnitt (31) zum Umgeben des Biopsats (20) für das Positionieren des Biopsieinstruments (10) in dem Verbindungsabschnitt (30) des Bergeelements (17) aufnehmbar, insbesondere einsteckbar, einrollbar und/oder einfaltbar, ist.

13. Kryobiopsiesonde (10) nach einem der vorstehenden Ansprüche, mit einem Sicherungselement (65) zur Sicherung des Bergeelements (17) gegen Umstülpen.

## Claims

1. Cryobiopsy probe comprising a biopsate recovery device (16) for recovering a biopsate (20) through an opening (46) with a recovery element (17) for fastening to or adjacent to a working tip (13) of the cryobiopsyprobe,
wherein the recovery element (17) is adapted to the folded over the biopsate (20) upon retraction of the working tip (13) through the opening (46), in order to enclose the biopsate (20) upon retraction of the working tip (13) through the opening (46), so as to separate the biopsate (20) from the environment upon the retraction through the opening (46),
wherein the recovery element (17) comprises, in longitudinal direction (L) of the recovery element (17), sequential sections (18, 30, 31) of the recovery element (17), wherein the recovery element (17) comprises a fastening section (18), a section (31) for enclosing the biopsate and a connecting section (30) that connects the section (31) for enclosing the biopsate (20) to the fastening section (18),
wherein the wall thickness (wu) of the section (31) for enclosing the biopsate (20) is less than the wall thickness (wv) of the connecting section (30).

2. Cryobiopsy probe according to claim 1, wherein the recovery element (17) is adapted to enclose the biopsate (20) in a circumferentially closed manner in order to fluidically separate the biopsate (20) from the environment.

3. Cryobiopsy probe according to one of the previous claims, wherein the recovery element (17) is provided with a structure adapted to engage the wall around the opening during retraction of the working tip in order to assist in folding the recovery element (17) over the biopsate when moving the biopsy instrument (10) back through the opening (46).

4. Cryobiopsy probe according to one of the previous claims, wherein the recovery element (17) comprises a support element (51) which is elastically deformable transversely to the retraction direction (ZR) and is adapted to bear against a wall (41) at the opening (46) during retraction.

5. Cryobiopsy probe according to one of the previous claims, wherein the recovery element (17) consists of a material or has a coating, which material or coating is adapted or selected to prevent freezing of the recovery element (17) to the biopsy instrument (10) and/or freezing of the biopsate (20) to the recovery element (17).

6. Cryobiopsy probe according to one of the previous claims, wherein the recovery element (17) has, adjoining or adjacent to the fastening side of the recovery element (17) to the biopsy instrument (10), a section (30) having an outer diameter widening along a longitudinal section of the biopsy instrument (10).

7. Cryobiopsy probe according to one of the previous claims, wherein the recovery element (17) is adapted to elastically expand to at most five times the length, particularly at most 1.5 times the length, of the unexpanded recovery element (17) while puncturing an organ wall (41).

8. Cryobiopsy probe according to one of the previous claims, wherein the wall of the recovery element (17) has a thickness of 0.005 mm to 2 mm inclusive, preferably less than 0.5 mm.

9. Cryobiopsy probe according to one of the previous claims, wherein the materials, of which the sequential sections consist, differ from section to section.

10. Cryobiopsy probe according to one of the previous claims, wherein the recovery element (17) has a surface non-adherent to cells and/or tissue.

11. Cryobiopsy probe (10) according to one of the previous claims with a working tip (13), wherein the working tip (13) is adapted to puncture an organ wall (41) with the working tip (13).

12. Cryobiopsy probe (10) according to one of the previous claims, wherein the section (31) for enclosing the biopsate (20) is receivable, in particular insertable, rollable and/or foldable, in a connecting section (30) of the recovery element (17) for positioning the biopsate instrument (10).

13. Cryobiopsyprobe according to one of the previous claims, with a securing element (65) for securing the recovery element (17) against folding over.

## Revendications

1. Sonde de cryobiopsie comprenant un dispositif de récupération de matériel biopsié (16) destiné à récupérer un matériel biopsié (20) à travers une ouverture (46), à l'aide d'un élément de récupération (17) destiné à être fixé sur ou à proximité d'une pointe de travail (13) de la sonde de cryobiopsie, l'élément de récupération (17) étant conçu pour être retourné sur le matériel biopsié (20) lorsque la pointe de travail (13) est retirée à travers l'ouverture (46), afin d'entourer le matériel biopsié (20) lors du retrait de la pointe de travail (13) à travers l'ouverture (46), afin de séparer le matériel biopsié (20) de l'environnement lors du retrait à travers l'ouverture (46), l'élément de récupération (17) présentant des segments (18, 30, 31) de l'élément de récupération (17) qui sont disposés les uns à la suite des autres dans le sens longitudinal (L) de l'élément de récupération (17), l'élément de récupération (17) présentant un segment de fixation (18), un segment (31) destiné à entourer le matériel biopsié, et un segment de liaison (30) qui relie le segment (31), destiné à entourer le matériel biopsié (20), au segment de fixation (18), l'épaisseur de paroi (wu) du segment (31) destiné à entourer le matériel biopsié (20) étant inférieure à l'épaisseur de paroi (wv) du segment de liaison (30).

2. Sonde de cryobiopsie selon la revendication 1, dans laquelle l'élément de récupération (17) est conçu pour entourer le matériel biopsié (20) sur son pourtour, afin de séparer le matériel biopsié (20) de l'environnement du point de vue des fluides.

3. Sonde de cryobiopsie selon l'une des revendications précédentes, dans laquelle l'élément de récupération (17) présente une structuration qui est conçue pour entrer en prise avec la paroi entourant l'ouverture, lors du retrait de la pointe de travail, en vue de favoriser le retournement de l'élément de récupération (17) sur le matériel biopsié (20) lors du déplacement en arrière de l'instrument de biopsie (10) à travers l'ouverture (46).

4. Sonde de cryobiopsie selon l'une des revendications précédentes, dans laquelle l'élément de récupération (17) présente un élément d'appui (51) qui peut être déformé de façon élastique transversalement à la direction de retrait (ZR) et qui est conçu pour s'appuyer sur une paroi (41) sur l'ouverture (46) lors du retrait.

5. Sonde de cryobiopsie selon l'une des revendications précédentes, dans laquelle l'élément de récupération (17) est constitué d'un matériau ou présente un revêtement, lequel matériau ou revêtement est conçu ou choisi pour empêcher que l'élément de récupération (17) ne gèle sur l'instrument de biopsie (10) et/ou que le matériel biopsié (20) ne gèle sur l'élément de récupération (17).

6. Sonde de cryobiopsie selon l'une des revendications précédentes, dans laquelle l'élément de récupération (17) présente, de façon adjacente ou à proximité du point de fixation de l'élément de récupération (17) sur l'instrument de biopsie (10), un segment (30) ayant un diamètre extérieur qui s'élargit le long d'une partie longitudinale de l'instrument de biopsie (10).

7. Sonde de cryobiopsie selon l'une des revendications précédentes, dans laquelle l'élément de récupération (17) est conçu pour que l'élément de récupération (17) s'allonge élastiquement au maximum à cinq fois la longueur, de manière particulièrement avantageuse à au maximum 1,5 fois la longueur de l'élément de récupération (17) non allongé, lors du perçage d'une paroi d'organe (41).

8. Sonde de cryobiopsie selon l'une des revendications précédentes, dans laquelle la paroi de l'élément de récupération (17) présente une épaisseur allant de 0,005 mm à 2 mm inclus, de préférence inférieure à 0,5 mm.

9. Sonde de cryobiopsie selon l'une des revendications précédentes, dans laquelle les matériaux constituant des segments disposés les uns à la suite des autres sont différents d'un segment à l'autre.

10. Sonde de cryobiopsie selon l'une des revendications précédentes, dans laquelle l'élément de récupération (17) présente une surface n'adhérant pas aux cellules et/ou aux tissus.

11. Sonde de cryobiopsie (10) selon l'une des revendications précédentes 1 comprenant une pointe de travail (13), la pointe de travail (13) étant réalisée pour percer une paroi d'organe (41) avec la pointe de travail (13).

12. Sonde de cryobiopsie (10) selon l'une des revendications précédentes, dans laquelle le segment (31), destiné à entourer le matériel biopsié (20), peut être logé, notamment enfiché, enroulé et/ou replié, dans le segment de liaison (30) de l'élément de récupération (17), en vue du positionnement de l'instrument de biopsie (10).

13. Sonde de cryobiopsie (10) selon l'une des revendications précédentes, comprenant un élément de protection (65) destiné à empêcher le retournement de l'élément de récupération (17).
